# EUROPEAN PATENT SPECIFICATION

(11) **EP 1 066 373 B1**
(45) Date of publication and mention of the grant of the patent: **14.01.2009**
(21) Application number: 99914442.1
(22) Date of filing: 22.03.1999
(51) Int. Cl.: C12N 9/16, C12N 15/55, A23K 1/165

(54) **PHYTASE VARIANTS**
VARIANTEN DER PHYTASE
VARIANT DE PHYTASE

(30) Priority: 23.03.1998 DK 40798; 19.06.1998 DK 80698; 18.09.1998 DK 117698; 22.01.1999 DK 9199
(43) Date of publication of application: 10.01.2001
(73) Proprietor: Novozymes A/S, 2880 Bagsvaerd (DK)
(72) Inventor: SVENDSEN, Allan, DK-2880 Bagsvaerd (DK); KOSTREWA, Dirk, D-79100 Freiburg (DE); LASSEN, Soeren Flensted, DK-2100 Copenhagen (DK)
(86) International application number: PCT/DK1999/000153
(87) International publication number: WO 1999/049022

(56) References cited:
- EP-A1- 0 420 358
- EP-A2- 0 897 010
- EP-A2- 0 897 985
- WO-A1-91/14782
- WO-A1-97/35016
- WO-A2-97/48812

## Description

### FIELD OF THE INVENTION

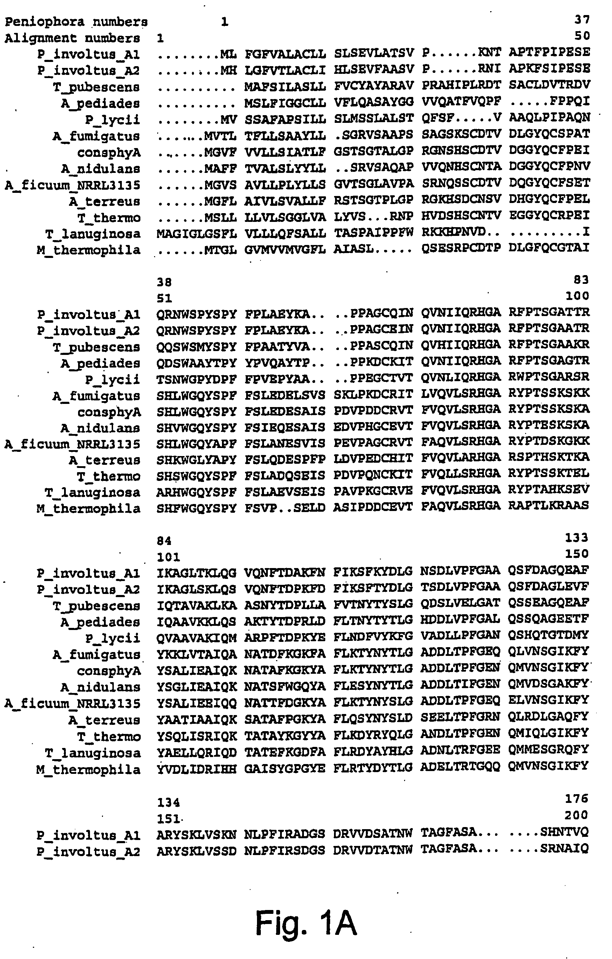

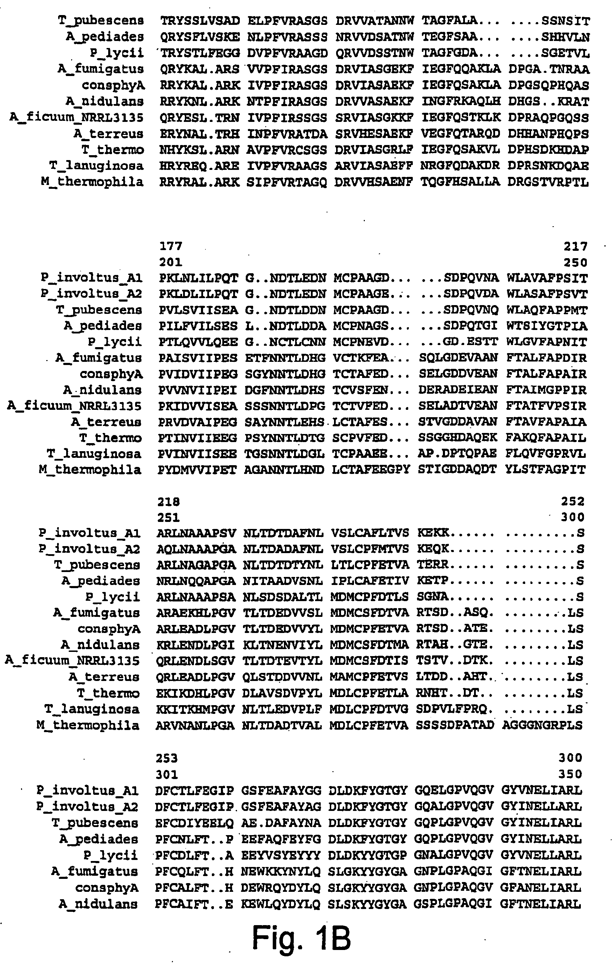

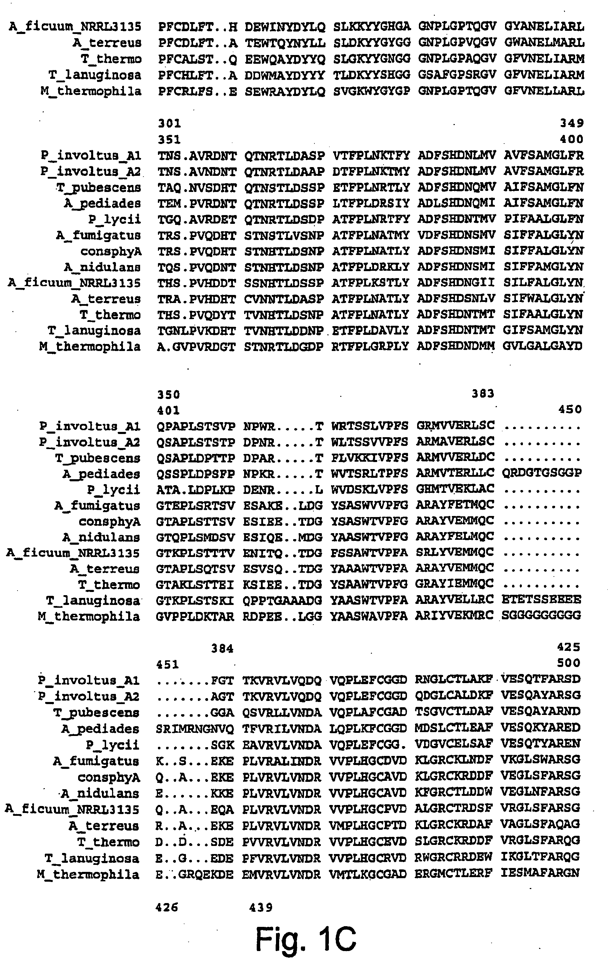

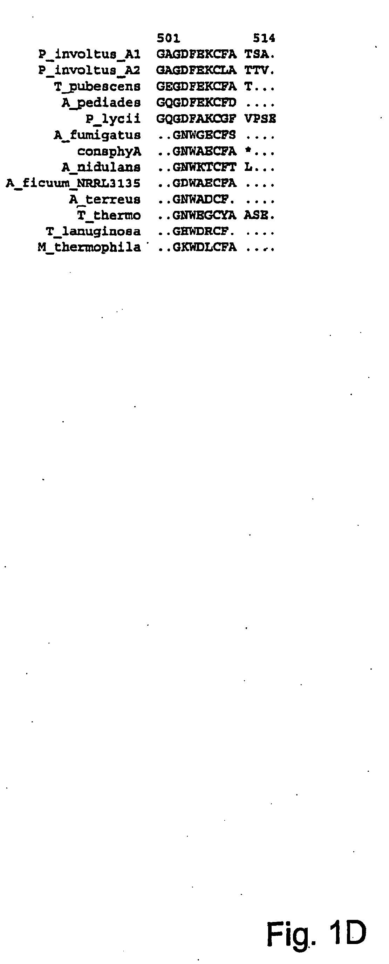

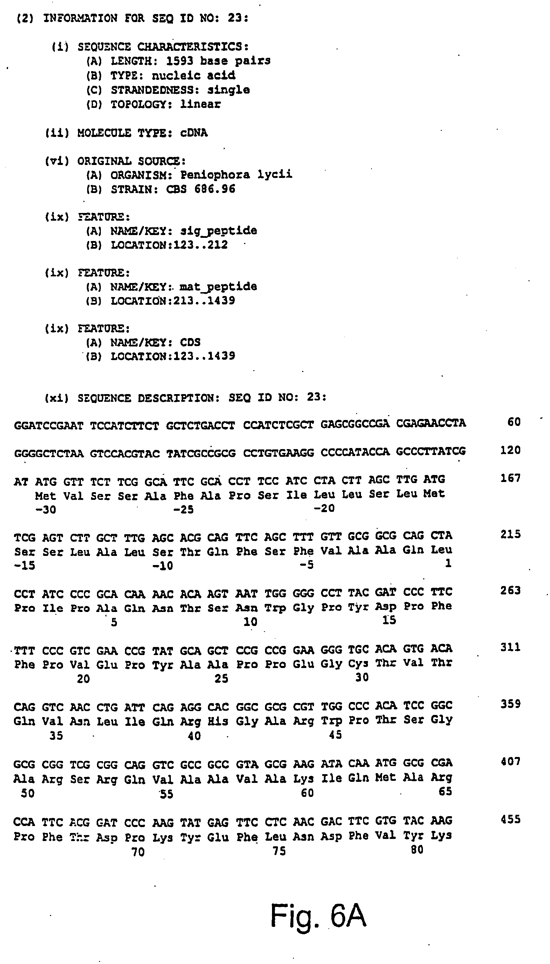

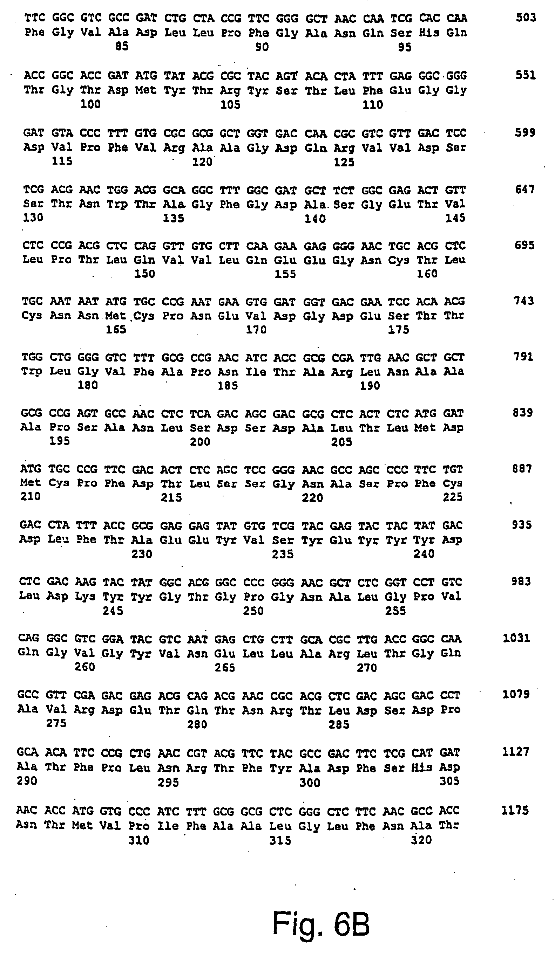

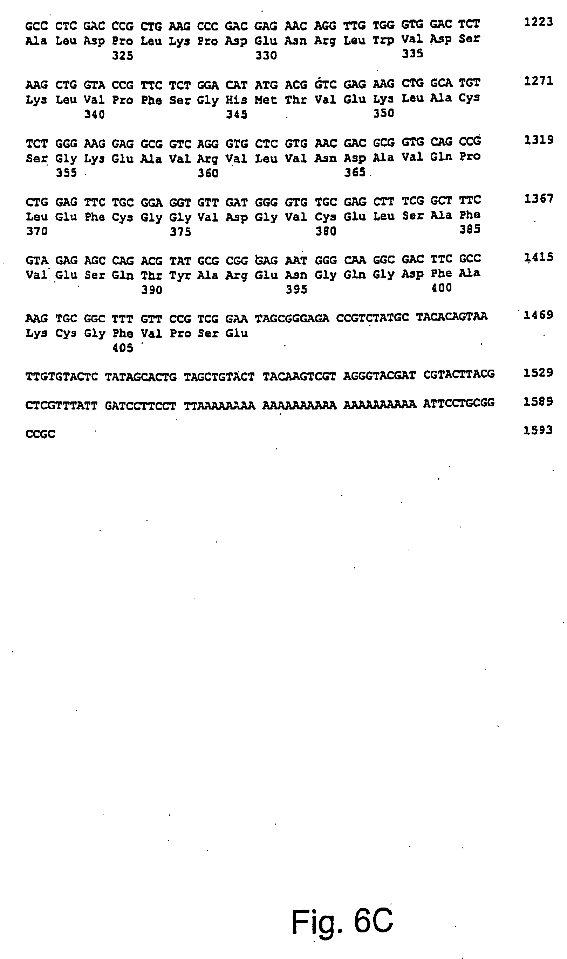

This invention relates to a phytase which differs from the Peniophora lycii phytase amino acid sequence of Fig. 6 in one or more of the following positions, using the position numbering corresponding to P_lycii of Fig. 1: D45S; T61R; A115N; L118V; H126S,V; G172P; E173Q,S; D201E; D203R,K,S; N215A, P; M238L; V264R, I; G302R, H; T337Q, S, G; V339I; P340A; E360R; V365L,A,S; D366V,S; E411K,T., the corresponding cloned DNA sequences, a method of producing such phytase variants, and the use thereof for a number of industrial applications.

### BACKGROUND OF THE INVENTION

Phytic acid or myo-inositol 1,2,3,4,5,6-hexakis dihydrogen phosphate (or for short myo-inositol hexakisphosphate) is the primary source of inositol and the primary storage form of phosphate in plant seeds. Phytin is a mixed potassium, magnesium and calcium salt of inositol.

The phosphate moieties of phytic acid chelates divalent and trivalent cations such as metal ions, i.a. the nutritionally essential ions of calcium, iron, zinc and magnesium as well as the trace minerals manganese, copper and molybdenum.

Phytic acid and its salts, phytates, are often not metabolized, i.e. neither the phosphorous thereof, nor the chelated metal ions are nutritionally available.

Accordingly, food and feed preparations need to be supplemented with inorganic phosphate and often also the nutritionally essential ions such as iron and calcium, must be supplemented.

Still further, the phytate phosphorus passes through the gastrointestinal tract of such animals and is excreted with the manure, resulting in an undesirable phosphate pollution of the environment resulting e.g. in eutrophication of the water environment and extensive growth of algae.

Phytic acid or phytates, said terms being, unless otherwise indicated, in the present context used synonymously or at random, are degradable by phytases.

The production of phytases by plants as well as by microorganisms has been reported. Amongst the microorganisms, phytase producing bacteria as well as phytase producing fungi are known.

There are several descriptions of phytase producing filamentous fungi belonging to the fungal phylum of Ascomycota (ascomycetes). In particular, there are several references to phytase producing ascomycetes of the Aspergillus genus such as Aspergillus terreus (Yamada et al., 1986, Agric. Biol. Chem. 322:1275-1282). Also, the cloning and expression of the phytase gene from Aspergillus niger var. awamori has been described (Piddington et al., 1993, Gene 133:55-62). EP 0420358 describes the cloning and expression of a phytase of Aspergillus ficuum (niger). EP 0684313 describes the cloning and expression of phytases of the ascomycetes Aspergillus niger, Myceliophthora thermophila, Aspergillus terreus. Still further, some partial sequences of phytases of Aspergillus nidulans, Talaromyces thermophilus, Aspergillus fumigatus and another strain of Aspergillus terreus are given.

The cloning and expression of a phytase of Thermomyces lanuginosus is described in WO 97/35017.

There is a current need for phytases of amended properties or characteristics, e.g. phytases of increased thermostability, altered pH optimum (a high pH optimum being desirable for in-vitro processing, a low for in-vivo processing in the gastro-intestinal tract), and/or of a higher specific activity.

### SUMMARY OF THE INVENTION

This invention relates to a phytase which differs from the Peniophora lycii phytase amino acid sequence of Fig. 6 in 5 one or more of the following positions, using the position numbering corresponding to P_lycii of Fig. 1: D45S; T61R; A115N; L118V; H126S,V; G172P; E173Q,S; D201E; D203R,K,S; N215A,P; M238L; V264R,I; G302R,H; T337Q,S,G; V339I; P340A; E360R; V365L,A,S; D366V,S; E411K,T., the corresponding cloned 10 DNA sequences, a method of producing such phytase variants, and the use thereof for a number of industrial applications.

### BRIEF DESCRIPTION OF THE DRAWINGS

In the detailed description of the invention below, reference is made to the drawings, of which
- Fig. 1: is an alignment of thirteen specific phytase sequences (a multiple sequence alignment according to the program PileUp; GapWeight: 3.000; GapLengthWeight: 0.100);
- Fig. 2: this figure shows the amino acid and DNA sequence of a first phytase ("P_involtus-A1") derived from strain CBS 100231 of Paxillus involutus which was deposited on 28.11.97; the expression plasmid pYES 2.0 comprising the full length cDNA sequence was transformed into E. coli strain DSM 11842 which was deposited on 12.11.97 (see WO 98/28409);
- Fig. 3: this figure shows the amino acid and DNA sequence of a second phytase ("P_involtus-A2") derived from strain CBS 100231 of Paxillus involutus which was deposited on 28.11.97; the expression plasmid pYES 2.0 comprising the full length cDNA sequence was transformed into E. coli strain DSM 11843 which was deposited on 12.11.97 (see WO 98/28409);
- Fig. 4: this figure shows the amino acid and DNA sequence of a phytase ("T_pubescens") derived from strain CBS 100232 of Trametes pubescens, which was deposited on 28.11.97; the expression plasmid pYES 2.0 comprising the full length cDNA sequence was transformed into E. coli strain DSM 11844 which was deposited on 12.11.97 (see WO 98/28409);

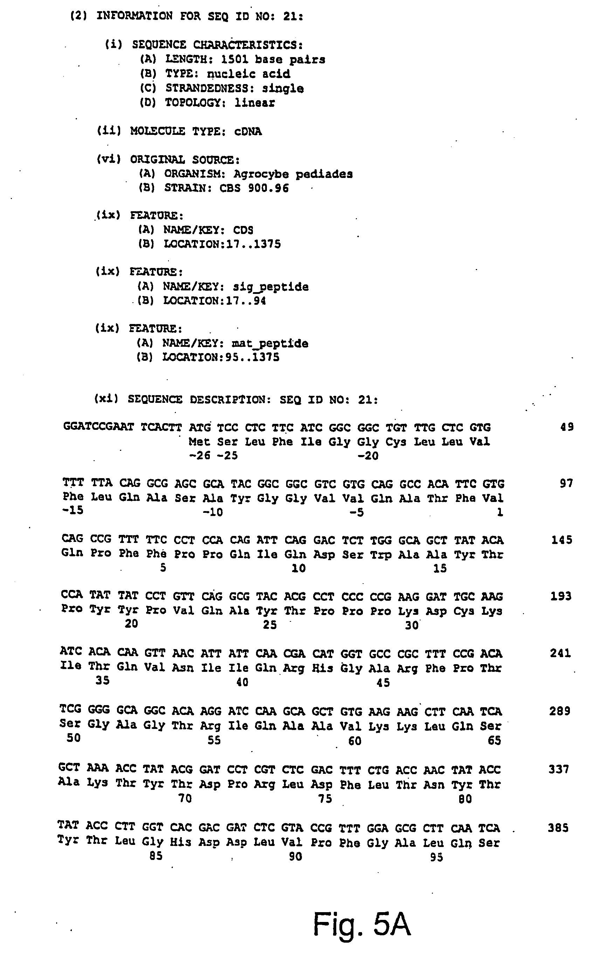

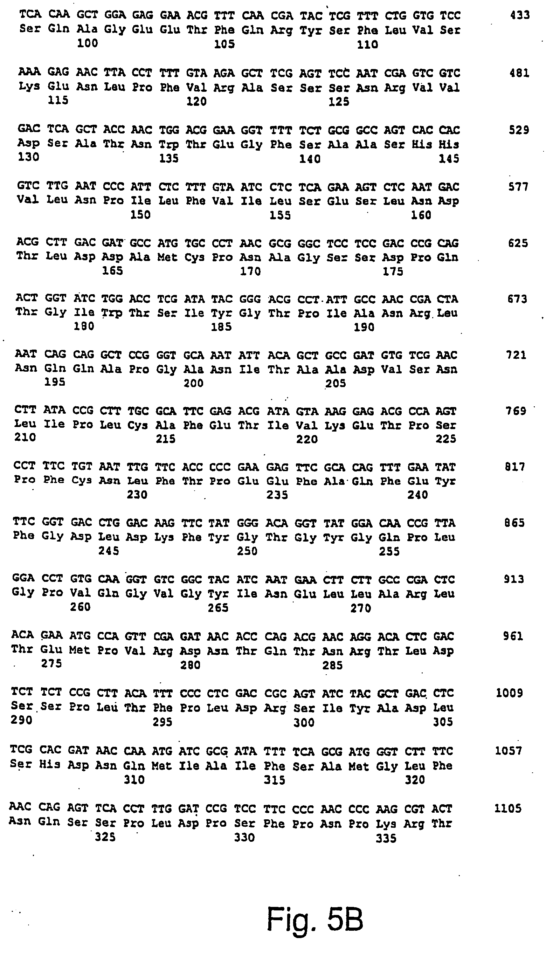

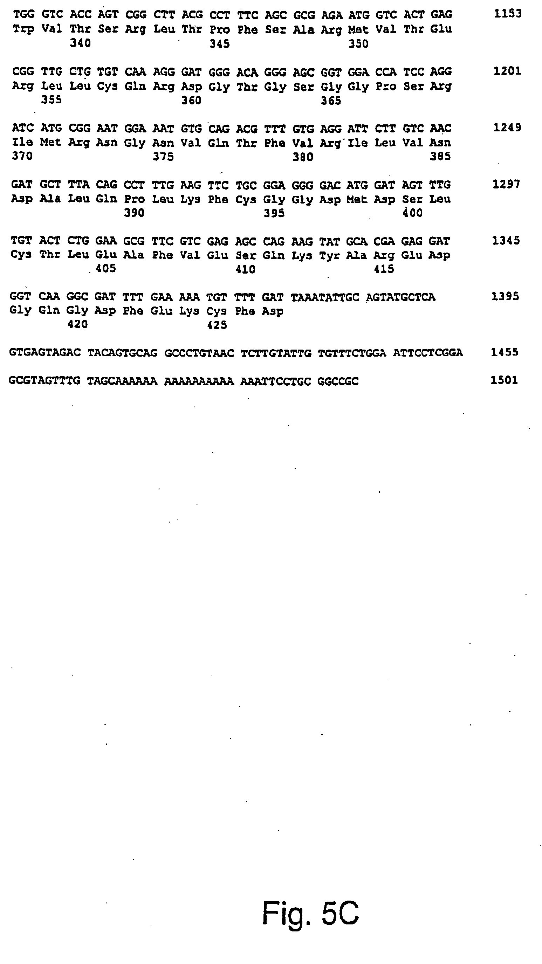

- Fig. 5: this figure shows the amino acid and DNA sequence of a phytase ("A_pediades") derived from strain CBS 900.96 of Agrocybe pediades deposited on 04.12.96; the expression plasmid pYES 2.0 comprising the full length cDNA sequence was transformed into E. coli strain DSM 11313 which was deposited on 02.12.96 (see WO 98/28409);
- Fig. 6: this figure shows the amino acid and DNA sequence of a phytase ("P_lycii") derived from strain CBS 686.96 of Peniophora lycii which was deposited on 04.12.96; the expression plasmid pYES 2.0 comprising the full length cDNA sequence was transformed into E. coli strain DSM 11312 which was deposited on 02.12.96 (see WO 98/28409);
- Fig. 7: this figure equals figure 2 of EP 0684313 and shows the amino acid and DNA sequence of a phytase ("M_thermophila") derived from strain ATCC 48102 (=ATCC 74340) of Myceliophthora thermophila which was re-deposited on 14.03.97;

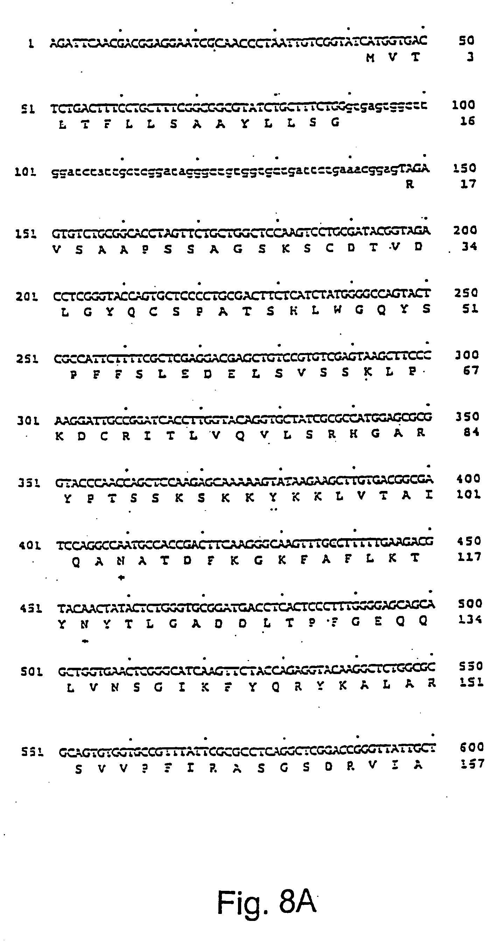

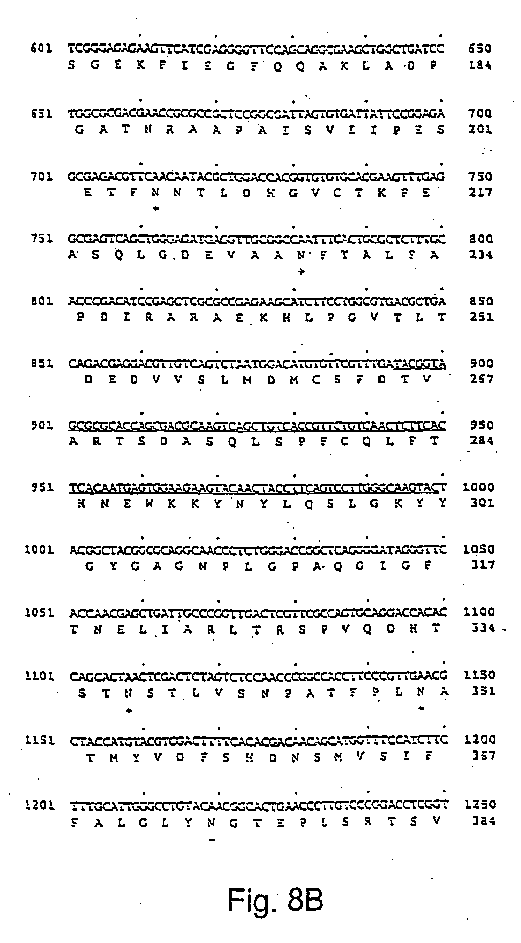

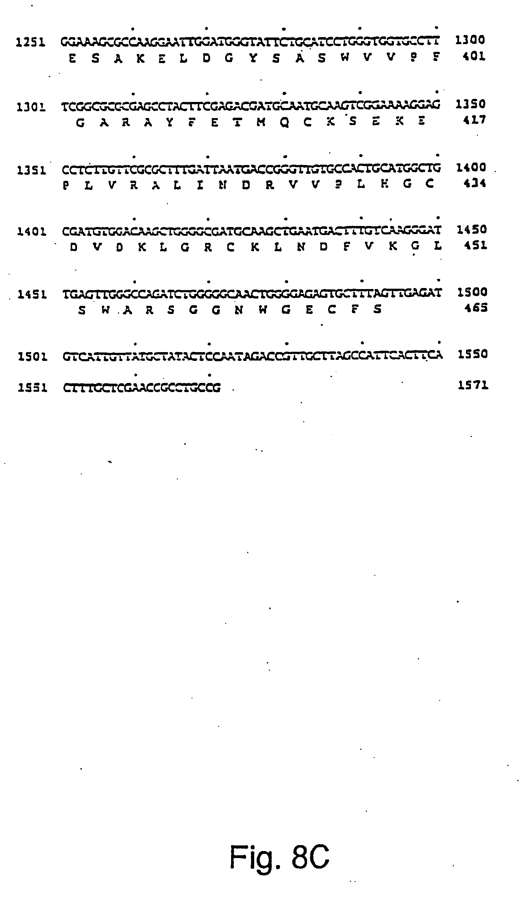

- Fig. 8: this figure shows the amino acid and DNA sequence of a phytase ("A fumigatus") derived from strain ATCC 13073 of Aspergillus fumigatus (see EP 0897985);
- Fig. 9: this figure shows the amino acid ("Conphys") and DNA sequence of an ascomycete consensus phytase (in the present context called "consphyA") (see EP 0897985);
- Fig. 10: this figure shows the amino acid and DNA sequence of a phytase ("A nidulans") derived from strain DSM 9743 of Aspergillus nidulans (see EP 0897985);

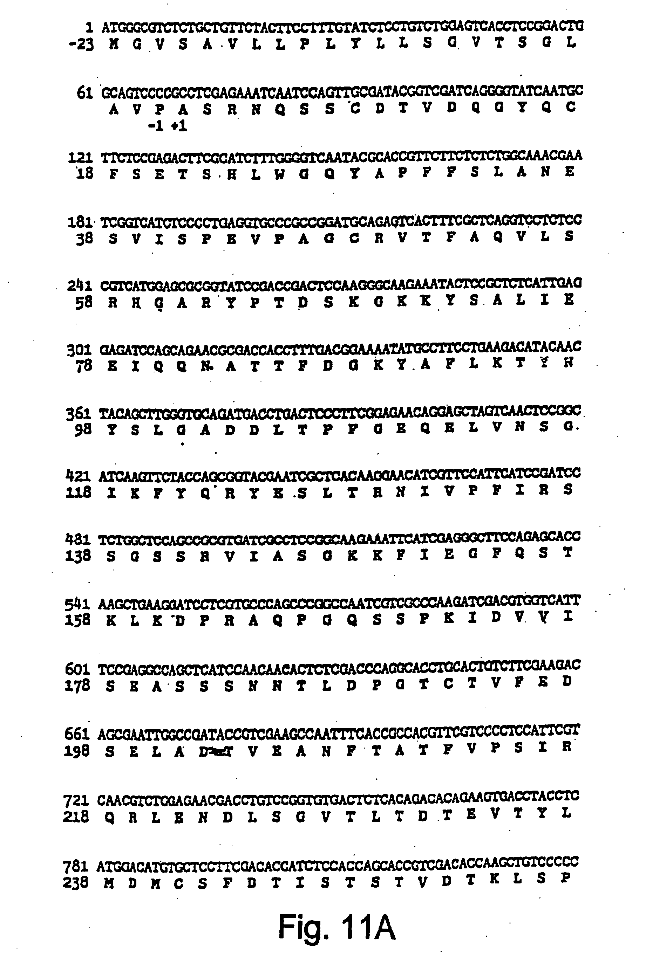

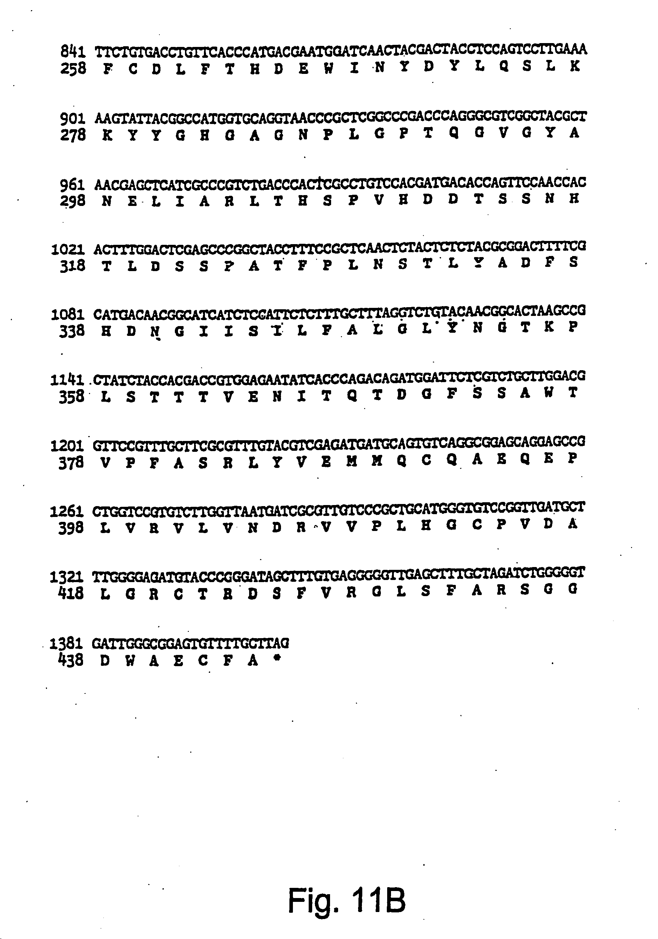

- Fig. 11: this figure equals figure 8 of EP 0420358 and shows the amino acid and DNA sequence of a phytase ("A_ficuum") derived from Aspergillus ficuum strain NRRL-3135;
- Fig. 12: this figure equals figure 1 of EP 0684313 and shows the amino acid and DNA sequence of a phytase ("A_terreus") derived from strain CBS 220.95 of Aspergillus terreus;
- Fig. 13: this figure shows the amino acid and DNA sequence of a phytase ("T_thermo") derived from strain ATCC 20186 (=ATCC 74338) of Talaromyces thermophilus which was redeposited on 14.03.97 (see EP 0897985);
- Fig. 14: this figure equals figure 2 of WO 97/35017 and shows the amino acid and DNA sequence of a phytase ("T_lanuginosa") derived from strain CBS 586.94 of Thermomyces lanuginosus; a plasmid comprising the full length cDNA sequence was transformed into E. coli DH5α (pMWR46) strain B-21527 which was deposited with NRRL on 23.02.96;
- Fig. 15: this figure shows the amino acid and DNA sequence of a phytase ("C_foecundissimum") derived from strain CBS 427.97 of Cladorrhinum foecundissimum which was deposited on 23 January 1997; the expression plasmid pYES 2.0 comprising the full length cDNA sequence was transformed into E. coli strain DSM 12742 which was deposited on 17 March 1999;
- Fig. 16: this figure shows an alignment of the phytase C_foecundissimum with the model phytase M_thermophila, using the program GAP gcg (Gap Weight 3.000; Length Weight 0.100); and
- Fig. 17: shows how the C_foecundissimum phytase can be pasted onto the alignment of Fig. 1.

### DETAILED DISCLOSURE OF THE INVENTION

### Phytase

In the present context a phytase is an enzyme which catalyzes the hydrolysis of phytate (myo-inositol hexakisphosphate) to (1) myo-inositol and/or (2) mono-, di-, tri-, tetra- and/or penta-phosphates thereof and (3) inorganic phosphate. In the following, for short, the above compounds are sometimes referred to as IP6, I, IP1, IP2, IP3, IP4, IP5 and P, respectively. This means that by action of a phytase, IP6 is degraded into P + one or more of the components IP5, IP4, IP3, IP2, IP1 and I. Alternatively, myo-inositol carrying in total n phosphate groups attached to positions p, q, r,.. is denoted Ins(p,q,r, ..)Pn. For convenience Ins (1, 2, 3, 4, 5, 6) P6 (phytic acid) is abbreviated PA.

According to the Enzyme nomenclature database ExPASy (a repository of information relative to the nomenclature of enzymes primarily based on the recommendations of the Nomenclature Committee of the International Union of Biochemistry and Molecular Biology (IUBMB) describing each type of characterized enzyme for which an EC (Enzyme Commission) number has been provided), two different types of phytases are known: A so-called 3-phytase (myo-inositol hexaphosphate 3-phosphohydrolase, EC 3.1.3.8) and a so-called 6-phytase (myo-inositol hexaphosphate 6-phosphohydrolase, EC 3.1.3.26). The 3-phytase hydrolyses first the ester bond at the D-3-position, whereas the 6-phytase hydrolyzes first the ester bond at the D-6- or L-6-position.

The expression "phytase" or "polypeptide or enzyme exhibiting phytase activity" is intended to cover any enzyme capable of effecting the liberation of inorganic phosphate or phosphorous from various myo-inositol phosphates. Examples of such myo-inositol phosphates (phytase substrates) are phytic acid and any salt thereof, e.g. sodium phytate or potassium phytate or mixed salts. Also any stereoisomer of the mono-, di- , tri-, tetra- or penta-phosphates of myo-inositol might serve as a phytase substrate. A preferred phytase substrate is phytic acid and salts thereof.

In accordance with the above definition, the phytase activity can be determined using any assay in which one of these substrates is used. In the present context (unless otherwise specified) the phytase activity is determined in the unit of FYT, one FYT being the amount of enzyme that liberates 1 µmol inorganic ortho-phosphate per min. under the following conditions: pH 5.5; temperature 37°C; substrate: sodium phytate (C₆H₆O₂₄P₆Na₁₂) in a concentration of 0.0050 mol/l. A suitable phytase assay is described in the experimental part.

The present invention provides a phytase as described in the appending claims.

A genetically engineered phytase is a non-naturally occuring phytase which is different from a model phytase, e.g. a wild-type phytase. Genetically engineered phytases include, but are not limited to, phytases prepared by site-directed mutagenesis, gene shuffling, random mutagenesis etc.

The invention also provides DNA constructs, vectors, host cells, and methods of producing these phytases, as well as uses thereof.

A phytase variant is a polypeptide or enzyme or a fragment thereof which exhibits phytase activity and which is amended as compared to a model phytase.

Amended means altered by way of one or more amino acid or peptide substitutions, deletions, insertions and/or additions - in each case by, or of, one or more amino acids. Such substitutions, deletions, insertions, additions can be achieved by any method known in the art, e.g. gene shuffling, random mutagenesis, site-directed mutagenesis etc.

The model or parent phytase, from which the phytase variant is derived, can be any phytase, e.g. a wild-type phytase or a derivative, mutant or variant thereof, including allelic and species variants, as well as genetically engineered variants thereof, which e.g. can be prepared by site-directed mutagenesis, random mutagenesis, shuffling etc.

Included in the concept of model phytase is also any hybrid or chimeric phytase, i.e. a phytase which comprises a combination of partial amino acid sequences derived from at least two phytases.

The hybrid phytase may comprise a combination of partial amino acid sequences deriving from at least two ascomycete phytases, at least two basidiomycete phytases or from at least one ascomycete and at least one basidiomycete phytase. These ascomycete and basidiomycete phytases from which a partial amino acid sequence derives may, e.g., be any of those specific phytases referred to herein.

In the present context, a hybrid, shuffled, random mutagenised, site-directed mutagenised or otherwise genetically engineered phytase derived from ascomycete phytases only is also an ascomycete phytase; and a hybrid, shuffled, random mutagenised, site-directed mutagenised or otherwise genetically engineered phytase derived from model basidiomycete phytases only is also a basidiomycete phytase. Any hybrid derived from at least one ascomycete phytase as well as at least one basidiomycete phytase is called a mixed ascomycete/basidiomycete phytase and such phytase is also a model phytase in the present context.

Analogously, a hybrid, shuffled, random mutagenised, site-directed mutagenised or otherwise genetically engineered phytase derived from one or more Aspergillus phytases is also an Aspergillus derived phytase; and a hybrid, shuffled, random mutagenised, site-directed mutagenised or otherwise genetically engineered phytase derived from any other taxonomic sub-grouping mentioned herein is also to be designated a phytase derived from this taxonomic sub-grouping.

Still further, in the present context, "derived from" is intended to indicate a phytase produced or producible by a strain of the organism in question, but also a phytase encoded by a DNA sequence isolated from such strain and produced in a host organism transformed with said DNA sequence. Finally, the term is intended to indicate a phytase which is encoded by a DNA sequence of synthetic and/or cDNA origin and which has the identifying characteristics of the phytase in question.

Preferably the model phytase is a phytase which can be aligned as described below to either of the thirteen phytases of Fig. 1 (which are particularly preferred model phytases).

Preferred wild-type model phytases (i.e. neither recombinant, or shuffled or otherwise genetically engineered phytases) have a degree of similarity or homology, preferably identity, to amino acid sequence no. 38-403 (Peniophora numbers) of either of these thirteen phytases of at least 40%, more preferably at least 50%, still more preferably at least 60%, in particular at least 70%, especially at least 80%, and in a most preferred embodiment a degree of similarity of at least 90%.

Preferred recombinant or shuffled or otherwise genetically engineered model phytases have a degree of similarity or homology, preferably identity, to partial sequence no. 38-49, 63-77, 274-291, 281-300 and 389-403 (Peniophora numbers) of either of these thirteen phytases of at least 60%, more preferably at least 70%, still more preferably at least 80%, in particular at least 90%.

The degree of similarity is preferably based on a comparison with the complete amino acid sequence of either of the thirteen phytases.

The degree of similarity or homology, alternatively identity, can be determined using any alignment programme known in the art. A preferred alignment programme is GAP provided in the GCG version 8 program package (Program Manual for the Wisconsin Package, Version 8, August 1994, Genetics Computer Group, 575 Science Drive, Madison, Wisconsin, USA 53711) (see also Needleman, S.B. and Wunsch, C.D., (1970), Journal of Molecular Biology, 48, 443-453). Using GAP with the following settings for polypeptide sequence comparison: GAP weight of 3.000 and GAP lengthweight of 0.100.

Also preferred is a wild-type model phytase which comprises an amino acid sequence encoded by a DNA sequence which hybridizes to a DNA sequence encoding amino acid sequence 38-403 (Peniophora numbers) of any of the DNA sequences encoding the thirteen specific phytase sequences of Fig. 1.

A further preferred model phytase is a genetically engineered phytase, which comprises an amino acid sequence encoded by a DNA sequence which hybridizes to a DNA sequence encoding amino acid sequence 38-49, and to a DNA sequence encoding amino acid sequence 63-77, and to a DNA sequence encoding amino acid sequence 274-291, and to a DNA sequence encoding amino acid sequence 281-300, and to a DNA sequence encoding amino acid sequence 389-403 (Peniophora numbers) of any of the DNA sequences encoding the thirteen specific phytase sequences of Fig. 1.

Preferably the hybridization is to the complete phytase encoding part of any of the thirteen phytases.

Suitable experimental conditions for determining whether a given DNA or RNA sequence "hybridizes" to a specified nucleotide or oligonucleotide probe involves presoaking of the filter containing the DNA fragments or RNA to examine for hybridization in 5 x SSC (Sodium chloride/Sodium citrate), (J. Sambrook, E.F. Fritsch, and T.. Maniatis, 1989, Molecular Cloning, A Laboratory Manual, 2d edition, Cold Spring Harbor, New York) for 10 min, and prehybridization of the filter in a solution of 5 x SSC, 5 x Denhardt's solution (Sambrook et al. 1989), 0.5 % SDS and 100 µg/ml of denatured sonicated salmon sperm DNA (Sambrook et al. 1989), followed by hybridization in the same solution containing a concentration of 10 ng/ml of a random-primed (Feinberg, A. P. and Vogelstein, B. (1983) Anal. Biochem. 132:6-13), 32P-dCTP-labeled (specific activity > 1 x 109 cpm/µg) probe for 12 hours at approximately 45°C.

The filter is then washed twice for 30 minutes in 2 x SSC, 0.5 % SDS at at least 55°C (low stringency), at at least 60°C (medium stringency), at at least 65°C (medium/high stringency), at at least 70°C (high stringency), or at at least 75°C (very high stringency).

Molecules to which the oligonucleotide probe hybridizes under these conditions are detected using an x-ray film.

It should be noted that a certain specific phytase variant need not actually have been prepared from a specific model phytase, for this model phytase to qualify as a "model phytase" in the present context. It is sufficient that the variant exhibits at least one of the herein indicated amendments when it is afterwards compared with the model phytase.

The alignment of Fig. 1 is made using the program PileUp (Program Manual for the Wisconsin Package, Version 8, August 1994, Genetics Computer Group, 575 Science Drive, Madison, Wisconsin, USA 53711), with a GapWeight of 3.000 and a GapLengthWeight of 0.100. When aligning a new model phytase or a new phytase variant all thirteen sequences can be included together with the new phytase (variant) in a multiple alignment, or, alternatively, at least one of the thirteen sequences of Fig. 1 is included together with the new phytase (variant) in an alignment.

A preferred procedure for aligning according to Fig. 1 a new model phytase (or a phytase variant) is as follows: The new model phytase is aligned with that specific sequence of the thirteen sequences of Fig. 1 to which the new model phytase has the highest degree of homology. For calculating the degree of homology, and for making the "alignment according to Fig. 1" of the two sequences, the program GAP referred to below is preferably used. Having aligned the two sequences, the new model phytase (or phytase variant) is added (pasted) to the alignment at Fig. 1 using the result of the first alignment (placing identical and homologous amino acid residues above each other as prescribed by the alignment), following which corresponding positions are now easily identifiable.

Example 7 shows an example of how to add a new model phytase to the alignment of Fig. 1 and deduce corresponding phytase variants thereof.

Other model phytases can be aligned and variants deduced in analogy with Example 7. This is so in particular for the following model phytases: The phytase of Aspergillus niger var. awamori (US patent no. 5,830,733); the Bacillus phytase of WO 98/06858; the soy bean phytase of WO 98/20139; the maize phytase of WO 98/05785; the Aspergillus phytase of WO 97/38096; the phytases of Monascus anka of WO 98/13480; the phytase from Schwanniomyces occidentalis of EP 0699762 etc.

When comparing a model phytase and a proposed phytase variant using the alignment as described herein, corresponding amino acid positions can be identified, viz. a model position of the model phytase and a variant position of the variant - the corresponding model position and variant position are simply placed one above the other in the alignment. An amendment is said to have occurred in a given position if the model amino acid of the model position and the variant amino acid of the variant position are different. Preferred amendments of these positions manifest themselves as amino acid substitutions, deletions or additions.

Amended in at least one position means amended in one or more positions, i.e. in one, two, three, four, five, six, seven, eight, nine, ten, eleven, twelve etc. up to all N positions listed. This definition includes any possible subcombinations thereof, e.g. any set of two substitutions, any set of three, any set of four, etc. - to any set of (N-1) positions.

In the present context all sequences, whatever the model phytase, and including the thirteen sequences of Fig. 1, are numbered using the numbering corresponding to the phytase P_lycii. These "Peniophora numbers" are indicated at Fig. 1, together with the "alignment numbers." The numbering of P_lycii starts at M1 and ends at E439.

As explained above, the alignment reveals which positions in various phytase sequences other than P_lycii are equivalent or corresponding to the given P. lycii position.

A substitution of amino acids is indicated herein as for instance "3S," which indicates, that at position 3 amino acid S should be substituted for the "original" or model position 3 amino acid, whichever it is. Thus, the substitution should result in an S in the corresponding variant position. Considering now the alignment at Fig. 1, a substitution like e.g. "3S" is to be interpreted as follows, for the respective phytases shown (the amino acid first indicated is the "original" or model amino acid in "Peniophora position" 3):

| | |
|---|---|
| P_involtus_A1: | F3S (number 3 F substituted by S) |
| P_involtus_A2: | L3S |
| T_pubescens: | M1S |
| A_pediades: | M1S |
| P_lycii: | redundant (already an S) |
| A_fumigatus: | T5S |
| consphyA: | V5S |
| A_nidulans: | T5S |
| A_ficuum_NRRL3135: | A5S |
| A_terreus: | A5S |
| T_thermo: | L5S |
| T_lanuginosa: | V11S |
| M_thermophila: | G5S |

However, in what follows the above specific substitutions will be designated as follows (always using the Peniophora numbering):

| | |
|---|---|
| P_involtus_A1: | F3S |
| P_involtus_A2: | L3S |
| T_pubescens: | M3S |
| A_pediades: | M3S |
| P_lycii: | redundant (already an S) |
| A_fumigatus: | T3S |
| consphyA: | V3S |
| A_nidulans: | T3S |
| A_ficuum_NRRL3135: | A3S |
| A_terreus: | A3S |
| T_thermo: | L3S |
| T_lanuginosa: | V3S |
| M_thermophila: | G3S |

Still further, denotations like e.g. "3S,F,G" means that the amino acid in position 3 (Peniophora numbers) of the model phytase in question is substituted with either of S, F or G, i.e. e.g. the designation "3S,F,G" is considered fully equivalent to the designation "3S, 3F, 3G".

A denotation like ()3S means that amino acid S is added to the sequence in question (at a gap in the actual sequence), in a position corresponding to Peniophora number 3 - and vice versa for deletions (S3()).

In case of regions in which the Peniophora phytase sequence has larger deletions than some of the other phytases in Fig. 1, for instance in the region between position 201 and 202 (Peniophora numbers), intermediate positions (amino acid residues in other sequences) are numbered by adding a,b,c,d, etc, in lower-case letters, to the last Peniophora position number, e.g. for the phytase M_thermophlla: E201; G201a; P201b; Y201c; S201d; T201e; I201f; G202; D203 etc.

In one of the priority applications of the present application there are two minor position numbering errors: According to the above definitions, the positions referred to in the first priority application as 204 and 205 (Peniophora numbers) are wrongly designated; they should have been numbered 203a and 204, respectively. Therefore, 204 has been substituted by 203a and 205 by 204 throughout the present application.

A preferred phytase variant comprises an amino acid sequence which comprises, preferably contains, one or more of the following amino acid substitutions: 24C; 27P; 31Y; 33C; 39H,S,Q; 40L,N; 42S, G; 43A, C, D, E, F, G, H, I, K, L, M, N, P, Q, R, S, T, V, W, Y; 44N; 45D, S; 47Y,F; 49P; 51E, A, R; 56P; 58D,K,A; 59G; 61R; 62V, I; 69Q; 75W, F; 78D, S; 79G; 80K, A; 81A, G, Q, E; 82T; 83A, I, K, R, Q; 84I, Y, Q, V; 88I; 90R, A; 102Y; 115N; 116S; 118V,L; 119E; 120L; 122A; 123N,Q,T; 125M,S; 126H,S,V; 127Q, E, N; 128A,S,T; 132F, I, L; 143N; 148V, I; 151A,S; 152G; 153D, Y; 154D, Q, S, G; 157V; 158D, A; 159T; 160A,S; 161T,N; 162N; 163W; 170fH; 170gA; 171N; 172P; 173Q,S; 184Q,S,P; 185S; 186A,E,P; 187A; 187aS; 190A,P; 193S; 194S, T; 195T, V, L; 198A,N,V; 200G,V; 201D,E; a deletion of at least one of 201a, 201b, 201c, 201d, 201e, 201f, preferably all; 201eT; 202S,A; 203R,K,S; 203aV,T; 204Q, E, S, A, V; 205E; 211L,V; 215A, P; 220L,N; 223H,D; 228N; 232T; 233E; 235Y, L, T; 236Y,N; 237F; 238L, M; 242P,S; 244D; 246V; 251eE, Q; 253P; 256D; 260A, H; 264R, I; 265A, Q; 267D; 270Y; A, L, G; 271D,N; 273D,K; 275F,Y; 278T, H; 280A, P; 283P; 287A,T; 288L, I, F; 292F,Y; 293A,V; 302R,H; 304P,A; 332F; 336S; 337T,G,Q,S; 338I; 339V, I; 340P,A; 343A, S, F, I, L; 348Y; 349P; 352K; 360R; 362P; 364W,F; 365V, L, A, S; 366D, S, V; 367A,K; 368K; 369I, L; 370V; 373A,S; 374S,A; 375H; 376M; 383kQ,E; 387P; 393V; 396R; 404A,G; 409R; 411K,T; 412R; 417E,R; 421F, Y; 431E.

Preferably this is with the proviso that the model phytase does not already comprise the above suggested amino acid substitution or addition or deletion at the position indicated. Or, with the proviso that, for each position, the model amino acid is not already the variant amino acid hereby proposed. But these provisos can be said to be in fact already inherent in the above wording, because of the expression "amended."

The various preferred phytase variants comprises, preferably contains or have, amino acid sequences which comprise or contain one or more of the amino acid substitutions, additions, or deletions listed in the respective claims.

The various phytase variants described herein comprise 1, 2, 3, 4, 5, 6, 7, 8, 9 or even 10 of these substitutions; or a number of substitutions of 10-15, 15-20, 20-30 or even 30-50; eventually up to 60, 70, 80 or 90 substitutions.

Preferably, the amino acid sequence of the various phytase variants comprise one or more substitutions of the substitution sub-groupings listed hereinbelow; or combinations of substitutions classified in two or more sub-groupings.

Generally, instead of "comprise," "contain" or "have," the amino acid sequences of preferred variants "consist essentially of" or "consist of" the specific model phytases of fig. 1, as modified by one or more of the substitutions described herein.

In the present context a basidiomycete means a microorganism of the phylum Basidiomycota. This phylum of Basidiomycota is comprised in the fungal kingdom together with e.g. the phylum Ascomycota ("ascomycetes").

Taxonomical questions can be clarified by consulting the references listed below or by consulting a fungal taxonomy database (NIH Data Base (Entrez)) which is available via the Internet on World Wide Web at the following address: http://www3.ncbi.nlm.nih.gov/Taxonomy/tax.html.

For a definition of basidiomycetes, reference is made to either Jülich, 1981, Higher Taxa of Basidiomycetes; Ainsworth & Bisby's (eds.) Dictionary of the Fungi, 1995, Hawksworth, D.L., P.M. Kirk, B.C. Sutton & D.N. Pegler; or Hansen & Knudsen (Eds.), Nordic Macromycetes, vol. 2 (1992) and 3 (1997). A preferred reference is Hansen & Knudsen.

For a definition of ascomycetes, reference is made to either of Ainsworth & Brisby cited above or Systema Ascomycetum by Eriksson, O.E. & D. L. Hawksworth, Vol. 16, 1998. A preferred reference is Eriksson et al.

Generally, a microorganism which is classified as a basidiomycete/ascomycete in either of the references listed above, including the database, is a basidiomycete/ascomycete in the present context.

Some Aspergillus strains are difficult to classify because they are anamorphous, and therefore they might be classified in Fungi Imperfecti. However, once the teleomorphous counterpart is found, it is re-classified taxonomically. For instance, the teleomorph of A. nidulans is Emericella nidulans (of the family Trichocomaceae, the order Eurotiales, the class Plectomycetes of the phylum Ascomycota). These subgroupings of Ascomycota are preferred, together with the family Lasiosphaeriaceae, the order Sordariales, the class Pyrenomycetes of the phylum Ascomycota.

The wording "ascomycetes" and analogues as used herein includes any strains of Aspergillus, Thermomyces, Myceliophthora, Talaromyces which are anamorphous and thus would be classified in Fungi Imperfecti.

Preferred basidiomycete phytases are those listed in WO 98/28409, in the very beginning of the section headed "Detailed description of the invention".

DNA sequences encoding the thirteen specifically listed model phytases and other model phytases can be prepared according to the teachings of each of the documents listed under the brief description of the drawings.

A DNA sequence encoding a model phytase may be isolated from any cell or microorganism producing the phytase in question, using various methods well known in the art. First, a genomic DNA and/or cDNA library should be constructed using chromosomal DNA or messenger RNA from the organism that produces the phytase. Then, if the amino acid sequence of the phytase is known, homologous, labelled oligonucleotide probes may be synthesized and used to identify phytase-encoding clones from a genomic library prepared from the organism in question. Alternatively, a labelled oligonucleotide probe containing sequences homologous to a known phytase gene could be used as a probe to identify phytase-encoding clones, using hybridization and washing conditions of lower stringency.

Yet another method for identifying phytaseencoding clones would involve inserting fragments of genomic DNA into an expression vector, such as a plasmid, transforming phytase-negative bacteria with the resulting genomic DNA library, and then plating the transformed bacteria onto agar containing a substrate for phytase thereby allowing clones expressing the phytase to be identified.

Alternatively, the DNA sequence encoding the enzyme may be prepared synthetically by established standard methods, e.g. the phosphoroamidite method described by S.L. Beaucage and M.H. Caruthers (1981) or the method described by Matthes et al. (1984). In the phosphoroamidite method, oligonucleotides are synthesized, e.g. in an automatic DNA synthesizer, purified, annealed, ligated and cloned in appropriate vectors.

Finally, the DNA sequence may be of mixed genomic and syn-thetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al. (1988).

DNA encoding the phytase variants can be prepared by methods known in the art, such as Site-directed Mutagenesis. Once a DNA sequence encoding a model phytase of interest has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the phytase-encoding sequence, is created in a vector carrying the phytase-encoding gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al. (1984). US 4,760,025 discloses the introduction of oligonucleotides encoding multiple mutations by performing minor alterations of the cassette. However, an even greater variety of mutations can be introduced at any one time by the Morinaga method because a multitude of oligonucleotides, of various lengths, can be introduced.

Another method of introducing mutations into DNA sequences encoding a desired model phytase is described in Nelson and Long (1989). It involves a 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

Yet another method of mutating DNA sequences encoding a model phytase is Random Mutagenesis. Random mutagenesis is suitably performed either as localised or region-specific random mutagenesis in at least three parts of the gene translating to the amino acid sequence shown in question, or within the whole gene.

The random mutagenesis of a DNA sequence encoding a model phytase may be conveniently performed by use of any method known in the art.

In relation to the above, a method for generating a variant of a model phytase is disclosed, wherein the variant preferably exhibits amended characteristics as described below, the method comprising:
(a) subjecting a DNA sequence encoding the model phytase to Site-directed Mutagenesis, or the Nelson and Long PCR mutagenesis method or to Random Mutagenesis,
(b) expressing the mutated DNA sequence obtained in step (a) in a host cell, and
(c) screening for host cells expressing a phytase variant which has an altered property relative to the model phytase.

When using Random Mutagenesis, step (a) of the above method of the invention is preferably performed using doped primers.

For instance, the random mutagenesis may be performed by use of a suitable physical or chemical mutagenizing agent, by use of a suitable oligonucleotide, or by subjecting the DNA sequence to PCR generated mutagenesis. Furthermore, the random mutagenesis may be performed by use of any combination of these mutagenizing agents. The mutagenizing agent may, e.g., be one which induces transitions, transversions, inversions, scrambling, deletions, and/or insertions.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues. When such agents are used, the mutagenesis is typically performed by incubating the DNA sequence encoding the parent enzyme to be mutagenized in the presence of the mutagenizing agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotide during the synthesis of the oligonucleotide at the positions which are to be changed. The doping or spiking may be done so that codons for unwanted amino acids are avoided. The doped or spiked oligonucleotide can be incorporated into the DNA encoding the phytase enzyme by any published technique, using e.g. PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints. The doping scheme may be made by using the DOPE program which, inter alia, ensures that introduction of stop codons is avoided.

When PCR-generated mutagenesis is used, either a chemically treated or non-treated gene encoding a model phytase is subjected to PCR under conditions that increase the mis-incorporation of nucleotides (Deshler 1992; Leung et al., Technique, Vol.1, 1989, pp. 11-15).

A mutator strain of E. coli (Fowler et al., Molec. Gen. Genet., 133, 1974, pp. 179-191), S. cereviseae or any other microbial organism may be used for the random mutagenesis of the DNA encoding the model phytase by, e.g., transforming a plasmid containing the parent glycosylase into the mutator strain, growing the mutator strain with the plasmid and isolating the mutated plasmid from the mutator strain. The mutated plasmid may be subsequently transformed into the expression organism.

The DNA sequence to be mutagenized may be conveniently present in a genomic or cDNA library prepared from an organism expressing the model phytase. Alternatively, the DNA sequence may be present on a suitable vector such as a plasmid or a bacteriophage, which as such may be incubated with or otherwise exposed to the mutagenising agent. The DNA to be mutagenized may also be present in a host cell either by being integrated in the genome of said cell or by being present on a vector harboured in the cell. Finally, the DNA to be mutagenized may be in isolated form. It will be understood that the DNA sequence to be subjected to random mutagenesis is pre-ferably a cDNA or a genomic DNA sequence.

In some cases it may be convenient to amplify the mutated DNA sequence prior to performing the expression step b) or the screening step c). Such amplification may be performed in accordance with methods known in the art, the presently preferred method being PCR-generated amplification using oligonucleotide primers prepared on the basis of the DNA or amino acid sequence of the parent enzyme.

Subsequent to the incubation with or exposure to the mutagenising agent, the mutated DNA is expressed by culturing a suitable host cell carrying the DNA sequence under conditions allowing expression to take place. The host cell used for this purpose may be one which has been transformed with the mutated DNA sequence, optionally present on a vector, or one which was carried the DNA sequence encoding the parent enzyme during the mutagenesis treatment. Examples of suitable host cells are the following: gram positive bacteria such as Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis, Streptomyces lividans or Streptomyces murinus; and gram-negative bacteria such as E. coli.

The mutated DNA sequence may further comprise a DNA sequence encoding functions permitting expression of the mutated DNA sequence.

The random mutagenesis may be advantageously localised to a part of the model phytase in question using Localized random mutagenesis. This may, e.g., be advantageous when certain regions of the enzyme have been identified to be of particular importance for a given property of the enzyme, and when modified are expected to result in a variant having improved properties. Such regions may normally be identified when the tertiary structure of the parent enzyme has been elucidated and related to the function of the enzyme.

The localized, or region-specific, random mutagenesis is conveniently performed by use of PCR generated mutagenesis techniques as described above or any other suitable technique known in the art. Alternatively, the DNA sequence encoding the part of the DNA sequence to be modified may be isolated, e.g., by insertion into a suitable vector, and said part may be subsequently subjected to mutagenesis by use of any of the mutagenesis methods discussed above.

For region-specific random mutagenesis with a view to amending e.g. the specific activity of a model phytase, codon positions corresponding to the following amino acid residues from the amino acid sequences set forth in Fig. 1 may appropriately be targeted:

Residues: 41-47, 68-80, 83-84, 115-118, 120-126, 128, 149-163, 184-185, 191-193, 198-201e, 202-203, 205, 235-236, 238-239, 242-243, 270-279, 285, 288, 332-343, 364-367, 369-375, 394.

Regions: 41-47, 68-80, 120-128, 149-163, 270-279, 332-343, 364-375.

The random mutagenesis may be carried out by the following steps:
1. Select regions of interest for modification in the parent enzyme
2. Decide on mutation sites and non-mutated sites in the selected region
3. Decide on which kind of mutations should be carried out, e.g. with respect to the desired stability and/or performance of the variant to be constructed
4. Select structurally reasonable mutations
5. Adjust the residues selected by step 3 with regard to step 4.
6. Analyse by use of a suitable dope algorithm the nucleotide distribution.
7. If necessary, adjust the wanted residues to genetic code realism, e.g. taking into account constraints resulting from the genetic code, e.g. in order to avoid introduction of stop codons; the skilled person will be aware that some codon combinations cannot be used in practice and will need to be adapted
8. Make primers
9. Perform random mutagenesis by use of the primers
10. Select resulting phytase variants by screening for the desired improved properties.

Suitable dope algorithms for use in step 6 are well known in the art. One such algorithm is described by Tomandl, D. et al., 1997, Journal of Computer-Aided Molecular Design 11:29-38. Another algorithm is DOPE (Jensen, LJ, Andersen, KV, Svendsen, A, and Kretzschmar, T (1998) Nucleic Acids Research 26:697-702).

A DNA sequence encoding a model phytase or a phytase variant can be expressed using an expression vector, a recombinant expression vector, which typically includes control sequences encoding a promoter, operator, ribosome binding site, translation initiation signal, and, optionally, a repressor gene or various activator genes.

The recombinant expression vector may be any vector which may conveniently be subjected to recombinant DNA procedures, and the choice of vector will often depend on the host cell into which it is to be introduced. Thus, the vector may be an autonomously replicating vector, e.g. a plasmid, a bacteriophage or an extra-chromosomal element. Alternatively, the vector may be one which, when introduced into a host cell, is integrated into the host cell genome and replicated together with the chromosome(s) into which it has been integrated.

In the vector, the DNA sequence should be operably connected to a suitable promoter sequence. The promoter may be any DNA sequence which shows transcriptional activity in the host cell of choice and may be derived from genes encoding proteins either homologous or heterologous to the host cell. An example of a suitable promoter for directing the transcription of the DNA sequence encoding a phytase variant of the invention, especially in a bacterial host, is the promoter of the lac operon of E.coli. For transcription in a fungal host, examples of useful promoters are those derived from the gene encoding A. oryzae TAKA amylase.

The expression vector of the invention may also comprise a suitable transcription terminator and, in eukaryotes, polyadenylation sequences operably connected to the DNA sequence encoding the phytase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the dal genes from B. subtilis or B. licheniformis, or one which confers antibiotic resistance such as ampicillin resistance. Furthermore, the vector may comprise Aspergillus selection markers such as amdS, argB, niaD and sC, or the selection may be accomplished by co-transformation, e.g. as described in WO 91/17243.

The procedures used to ligate the DNA construct of the invention encoding a phytase variant, the promoter, terminator and other elements, respectively, and to insert them into suitable vectors containing the information necessary for replication, are well known to persons skilled in the art (cf., for instance, Sambrook et al. (1989)).

The cell of the invention, either comprising a DNA construct or an expression vector of the invention as defined above, is advantageously used as a host cell in the recombinant production of a phytase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g. by homologous or heterologous recombination. Alternatively, the cell may be transformed with an expression vector as described above in connection with the different types of host cells.

An isolated DNA molecule or, alternatively, a "cloned DNA sequence" "a DNA construct," "a DNA segment" or "an isolated DNA sequence" refers to a DNA molecule or sequence which can be cloned in accordance with standard cloning procedures used in genetic engineering to relocate the DNA segment from its natural location to a different site where it will be replicated. The term refers generally to a nucleic acid sequence which is essentially free of other nucleic acid sequences, e.g., at least about 20% pure, preferably at least about 40% pure, more preferably about 60% pure, even more preferably about 80% pure, most preferably about 90% pure, and even most preferably about 95% pure, as determined by agarose gel electrophoresis. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The term "vector" is intended to include such terms/objects as "nucleic acid constructs," "DNA constructs," expression vectors" or "recombinant vectors."

The nucleic acid construct comprises a nucleic acid sequence of the present invention operably linked to one or more control sequences capable of directing the expression of the coding sequence in a suitable host cell under conditions compatible with the control sequences.

"Nucleic acid construct" is defined herein as a nucleic acid molecule, either single or double-stranded, which is isolated from a naturally occurring gene or which has been modified to contain segments of nucleic acid which are combined and juxtaposed in a manner which would not otherwise exist in nature.

The term nucleic acid construct may be synonymous with the term expression cassette when the nucleic acid construct contains all the control sequences required for expression of a coding sequence of the present invention.

The term "coding sequence" as defined herein primarily comprises a sequence which is transcribed into mRNA and translated into a polypeptide of the present invention when placed under the control of the above mentioned control sequences. The boundaries of the coding sequence are generally determined by a translation start codon ATG at the 5'-terminus and a translation stop codon at the 3'-terminus. A coding sequence can include, but is not limited to, DNA, cDNA, and recombinant nucleic acid sequences.

The term "control sequences" is defined herein to include all components which are necessary or advantageous for expression of the coding sequence of the nucleic acid sequence. Each control sequence may be native or foreign to the nucleic acid sequence encoding the polypeptide. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region' of the nucleic acid sequence encoding a polypeptide.

A "host cell" or "recombinant host cell" encompasses any progeny of a parent cell which is not identical to the parent cell due to mutations that occur during replication.

The cell is preferably transformed with a vector comprising a nucleic acid sequence of the invention followed by integration of the vector into the host chromosome.

"Transformation" means introducing a vector comprising a nucleic acid sequence of the present invention into a host cell so that the vector is maintained as a chromosomal integrant or as a self-replicating extra-chromosomal vector. Integration is generally considered to be an advantage as the nucleic acid sequence is more likely to be stably maintained in the cell. Integration of the vector into the host chromosome may occur by homologous or non-homologous recombination as described above.

The host cell may be a unicellular microorganism, e.g., a prokaryote, or a non-unicellular microorganism, e.g., a eukaryote. Examples of a eukaryote cell is a mammalian cell, an insect cell, a plant cell or a fungal cell. Useful mammalian cells include Chinese hamster ovary (CHO) cells, HeLa cells, baby hamster kidney (BHK) cells, COS cells, or any number of other immortalized cell lines available, e.g., from the American Type Culture Collection.

In a preferred embodiment, the host cell is a fungal cell.

Fungal cells may be transformed by a process involving protoplast formation, transformation of the protoplasts, and regeneration of the cell wall in a manner known per se.

The present invention also relates to a transgenic plant, plant part, such as a plant seed, or plant cell. Also compositions and uses of such plant or plant part are disclosed, especially its use as feed and food or additives therefore, along the lines of the present use and food/feed claims.

The transgenic plant can be dicotyledonous or monocotyledonous, for short a dicot or a monocot. Of primary interest are such plants which are potential food or feed components and which comprise phytic acid. A normal phytic acid level of feed components is 0.1-100 g/kg, or more usually 0.5-50 g/kg, most usually 0.5-20 g/kg. Examples of monocot plants are grasses, such as meadow grass (blue grass, Poa), forage grass such as festuca, lolium, temperate grass, such as Agrostis, and cereals, e.g. wheat, oats, rye, barley, rice, sorghum and maize (corn) .

Examples of dicot plants are legumes, such as lupins, pea, bean and soybean, and cruciferous (family Brassicaceae), such as cauliflower, oil seed rape and the closely related model organism Arabidopsis thaliana.

Such transgenic plant etc. is capable of degrading its own phytic acid, and accordingly the need for adding such enzymes to food or feed comprising such plants is alleviated. Preferably, the plant or plant part, e.g. the seeds, are ground or milled, and possibly also soaked before being added to the food or feed or before the use, e.g. intake, thereof, with a view to adapting the speed of the enzymatic degradation to the actual use.

If desired, the plant produced enzyme can also be recovered from the plant. In certain cases the recovery from the plant is to be preferred with a view to securing a heat stable formulation in a potential subsequent pelleting process.

Examples of plant parts are stem, callus, leaves, root, fruits, seeds, tubers etc. But also any plant tissue is included in this definition.

Any plant cell, whatever the tissue origin, is included in the definition of plant cells above.

Also included within the scope of the invention are the progeny of such plants, plant parts and plant cells.

The skilled man will know how to construct a DNA expression construct for insertion into the plant in question, paying regard i.a. to whether the enzyme should be excreted in a tissue specific way. Of relevance for this evaluation is the stability (pH-stability, degradability by endogenous proteases etc.) of the phytase in the expression compartments of the plant. He will also be able to select appropriate regulatory sequences such as promoter and terminator sequences, and signal or transit sequences if required (Tague et al, Plant, Phys., 86, 506, 1988).

The plant, plant part etc. can be transformed with this DNA construct using any known method. An example of such method is the transformation by a viral or bacterial vector such as bacterial species of the genus Agrobacterium genetically engineered to comprise the gene encoding the phytase of the invention. Also methods of directly introducing the phytase DNA into the plant cell or plant tissue are known in the art, e.g. micro injection and electroporation (Gasser et al, Science, 244, 1293; Potrykus, Bio/Techn. 8, 535, 1990; Shimamoto et al, Nature, 338, 274, 1989).

Following the transformation, the transformants are screened using any method known to the skilled man, following which they are regenerated into whole plants.

These plants etc. as well as their progeny then carry the phytase encoding DNA as a part of their genetic equipment.

In general, reference is made to WO 9114782A and WO 9114772A.

Agrobacterium tumefaciens mediated gene transfer is the method of choice for generating transgenic dicots (for review Hooykas & Schilperoort, 1992. Plant Mol. Biol. 19: 15-38), however it can also be used for transforming monocots. Due to host range limitations it is generally not possible to transform monocots with the help of A. tumefaciens. Here, other methods have to be employed. The method of choice for generating transgenic monocots is particle bombardment (microscopic gold or tungsten particles coated with the transforming DNA) of embryonic calli or developing embryos (Christou, 1992. Plant J. 2: 275-281; Shimamoto, 1994. Curr. Opin. Biotechnol. 5: 158-162; Vasil et al., 1992. Bio/Technology 10: 667-674).

Also other systems for the delivery of free DNA into these plants, including viral vectors (Joshi & Joshi, 1991. FEBS Lett. 281: 1-8), protoplast transformation via polyethylene glycol or electroporation (for review see Potyrkus, 1991. Annu. Rev. Plant Physiol. Plant Mol. Biol. 42: 205-225), microinjection of DNA into mesophyll protoplasts (Crossway et al., 1986. Mol. Gen. Genet. 202: 79-85), and macroinjection of DNA into young floral tillers of cereal plants (de la Pena et al., 1987. Nature 325: 274-276) are preferred methods.

In general, the cDNA or gene encoding the phytase variant is placed in an expression cassette (e.g. Pietrzak et al., 1986. Nucleic Acids Res. 14: 5857-5868) consisting of a suitable promoter active in the target plant and a suitable terminator (termination of transcription). This cassette (of course including a suitable selection marker, see below) will be transformed into the plant as such in case of monocots via particle bombardment. In case of dicots the expression cassette is placed first into a suitable vector providing the T-DNA borders and a suitable selection marker which in turn are transformed into Agrobacterium tumefaciens. Dicots will be transformed via the Agrobacterium harbouring the expression cassette and selection marker flanked by T-DNA following standard protocols (e.g. Akama et al., 1992. Plant Cell Reports 12: 7-11). The transfer of T-DNA from Agrobacterium to the Plant cell has been recently reviewed (Zupan & Zambryski, 1995. Plant Physiol. 107: 1041-1047). Vectors for plant transformation via Agrobacterium are commercially available or can be obtained from many labs that construct such vectors (e.g. Deblaere et al., 1985. Nucleic Acids Res. 13: 4777-4788; for review see Klee et al., 1987. Annu. Rev. Plant Physiol. 38: 467-486).

Available plant promoters: Depending on the process under manipulation, organ- and/or cell-specific expression as well as appropriate developmental and environmental control may be required. For instance, it is desirable to express a phytase cDNA in maize endosperm etc. The most commonly used promoter has been the constitutive 35S-CaMV promoter Franck et al., 1980. Cell 21: 285-294). Expression will be more or less equal throughout the whole plant. This promoter has been used successfully to engineer herbicide- and pathogen-resistant plants (for review see Stitt & Sonnewald, 1995. Annu. Rev. Plant Physiol. Plant Mol. Biol. 46: 341-368). Organ-specific promoters have been reported for storage sink tissues such as seeds, potato tubers, and fruits (Edwards & Coruzzi, 1990. Annu. Rev. Genet. 24: 275-303), and for metabolic sink tissues such as meristems (Ito et al., 1994. Plant Mol. Biol. 24: 863-878).

The medium used to culture the transformed host cells may be any conventional medium suitable for growing the host cells in question. The expressed phytase may conveniently be secreted into the culture medium and may be recovered therefrom by well-known procedures including separating the cells from the medium by centrifugation or filtration, precipitating proteinaceous com-ponents of the medium by means of a salt such as ammonium sulphate, followed by chromatographic procedures such as ion exchange chromatography, affinity chromatography, or the like.

Preferred host cells are a strain of Fusarium, Hansenula, Trichoderma or Aspergillus, in particular a strain of Fusarium graminearum, Fusarium venenatum, Fusarium cerealis, Fusarium sp. having the identifying characteristic of Fusarium ATCC 20334, as further described in PCT/US/95/07743, Hansenula polymorpha, Triahoderma harzianum or Trichoderma reesei, Aspergillus niger or Aspergillus oryzae.

References for expression in Hansenula polymorpha: Gellissen, G., Piontek, M., Dahlems, U., Jenzelewski, V., Gavagan, J.E., DiCosimo, R., Anton, D.I. & Janowicz, Z.A. (1996) Recombinant Hansenula polymorpha as a biocatalyst: coexpression of the spinach glycolate oxidase (GO) and the S. cerevisiae catalase T (CTT1) gene. Appl. Microbiol. Biotechnol. 46, 46-54.

Some more specific uses of the phytase variants appear from PCT/DK97/00568, the last pages of the detailed description of the invention section.

In a preferred embodiment, the phytase variant is essentially free of other non-phytase polypeptides, e.g., at least about 20% pure, preferably at least about 40% pure, more preferably about 60% pure, even more preferably about 80% pure, most preferably about 90% pure, and even most preferably about 95% pure, as determined by SDS-PAGE. Sometimes such polypeptide is alternatively referred to as a "purified" and/or "isolated" phytase.

A phytase polypeptide which comprises a phytase variant includes fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) encoding a phytase variant. Techniques for producing fusion polypeptides are known in the art, and include, ligating the coding sequences encoding the polypeptides so that they are in frame and that expression of the fused polypeptide is under control of the same promoter(s) and terminator.

A "feed" and a "food," respectively, means any natural or artificial diet, meal or the like or components of such meals intended or suitable for being eaten, taken in, digested, by an animal and a human being, respectively.

The phytase variant may exert its effect in vitro or in vivo, i.e. before intake or in the stomach of the individual, respectively. Also a combined action is possible.

A phytase composition according to the invention always comprises at least one phytase of the invention.

Generally, phytase compositions are liquid or dry.

Liquid compositions need not contain anything more than the phytase enzyme, preferably in a highly purified form. Usually, however, a stabilizer such as glycerol, sorbitol or mono propylen glycol is also added. The liquid composition may also comprise other additives, such as salts, sugars, preservatives, pH-adjusting agents, proteins, phytate (a phytase substrate). Typical liquid compositions are aqueous or oil-based slurries. The liquid compositions can be added to a food or feed after an optional pelleting thereof.

Dry compositions may be spray-dried compositions, in which case the composition need not contain anything more than the enzyme in a dry form. Usually, however, dry compositions are so-called granulates which may readily be mixed with e.g. food or feed components, or more preferably, form a component of a pre-mix. The particle size of the enzyme granulates preferably is compatible with that of the other components of the mixture. This provides a safe and convenient means of incorporating enzymes into e.g. animal feed.

Agglomeration granulates are prepared using agglomeration technique in a high shear mixer (e.g. Lödige) during which a filler material and the enzyme are co-agglomerated to form granules. Absorption granulates are prepared by having cores of a carrier material to absorb/be coated by the enzyme.

Typical filler materials are salts such as disodium sulphate. Other fillers are kaolin, talc, magnesium aluminium silicate and cellulose fibres. Optionally, binders such as dextrins are also included in agglomeration granulates.

Typical carrier materials are starch, e.g. in the form of cassava, corn, potato, rice and wheat. Salts may also be used.

Optionally, the granulates are coated with a coating mixture. Such mixture comprises coating agents, preferably hydrophobic coating agents, such as hydrogenated palm oil and beef tallow, and if desired other additives, such as calcium carbonate or kaolin.

Additionally, phytase compositions may contain other substituents such as colouring agents, aroma compounds, stabilizers, vitamins, minerals, other feed or food enhancing enzymes, i.e. enzymes that enhances the nutritional properties of feed/food, etc. This is so in particular for the so-called pre-mixes.

A "food or feed additive" is an essentially pure compound or a multi component composition intended for or suitable for being added to food or feed. In particular it is a substance which by its intended use is becoming a component of a food or feed product or affects any characteristics of a food or feed product. It is composed as indicated for phytase compositions above. A typical additive usually comprises one or more compounds such as vitamins, minerals or feed enhancing enzymes and suitable carriers and/or excipients.

In a preferred embodiment, the phytase composition of the invention additionally comprises an effective amount of one or more feed enhancing enzymes, in particular feed enhancing enzymes selected from the group consisting of α-galactosidases, β-galactosidases, in particular lactases, other phytases, β-glucanases, in particular endo-β-1,4-glucanases and endo-β-1,3(4)-glucanases, cellulases, xylosidases, galactanases, in particular arabinogalactan endo-1,4-β-galactosidases and arabinogalactan endo-1,3-β-galactosidases, endoglucanases, in particular endo-1,2-β-glucanase, endo-1,3-α-glucanase, and endo-1,3-β-glucanase, pectin degrading enzymes, in particular pectinases, pectinesterases, pectin lyases, polygalacturonases, arabinanases, rhamnogalacturonases, rhamnogalacturonan acetyl esterases, rhamnogalacturonan-α-rhamnosidase, pectate lyases, and α-galacturonisidases, mannanases, β-mannosidases, mannan acetyl esterases, xylan acetyl esterases, proteases, xylanases, arabinoxylanases and lipolytic enzymes such as lipases, phospholipases and cutinases.

The animal feed additive is supplemented to the mono-gastric animal before or simultaneously with the diet. Preferably, the animal feed additive is supplemented to the mono-gastric animal simultaneously with the diet. In a more preferred embodiment, the animal feed additive is added to the diet in the form of a granulate or a stabilized liquid.

An effective amount of phytase in food or feed is from about 10-20.000; preferably from about 10 to 15.000, more preferably from about 10 to 10.000, in particular from about 100 to 5.000, especially from about 100 to about 2.000 FYT/kg feed or food.

Examples of other specific uses of the phytase of the invention is in soy processing and in the manufacture of inositol or derivatives thereof.

The invention also relates to a method for reducing phytate levels in animal manure, wherein the animal is fed a feed comprising an effective amount of the phytase of the invention.

Also comprised in this invention is the use of a phytase of the invention during the preparation of food or feed preparations or additives, i.e. the phytase exerts its phytase activity during the manufacture only and is not active in the final food or feed product. This aspect is relevant for instance in dough making and baking.

Described herein is a phytase variant which, when aligned according to Fig. 1, is amended as compared to a model phytase in at least one of the following positions, using the position numbering corresponding to P_lycii: 24; 27; 31; 33; 39; 40; 41; 42; 43; 44; 45; 46; 47; 49; 51; 56; 58; 59; 61; 62; 68; 69; 70; 71; 72; 73; 74; 75; 76; 77; 78; 79; 80; 81; 82; 83; 84; 88; 90; 102; 115; 116; 117; 118; 119; 120; 121; 122; 123; 124; 125; 126; 127; 128; 132; 143; 148; 149; 150; 151; 152; 153; 154; 155; 156; 157; 158; 159; 160; 161; 162; 163; 170f; 170g; 171; 172; 173; 184; 185; 186; 187; 187a; . 190; 191; 192; 193; 194; 195; 198; 199; 200; 201; 201a; 201b; 201c; 201d; 201e; 201f; 202; 203; 203a; 204; 205; 211; 215; 220; 223; 228; 232; 233; 234; 235; 236; 237; 238; 239; 242; 243; 244; 246; 251e; 253; 256; 260; 264; 265; 267; 270; 271; 272; 273; 274; 275; 276; 277; 278; 279; 280; 283; 285; 287; 288; 292; 293; 302; 304; 332; 333; 334; 335; 336; 337; 338; 339; 340; 341; 342; 343; 348; 349; 352; 360; 362; 364; 365; 366; 367; 368; 369; 370; 371; 372; 373; 374; 375; 376; 383k; 387; 393; 394; 396; 404; 409; 411; 412; 413; 417; 421; 431.

From these variants we expect amended characteristics, preferably amended activity characteristics. In fact, for several variants such amended characteristics have already been shown (see the experimental part). Like above, "amended" means as compared to the model phytase. "Amended activity characteristics" means amended in at least one phytase activity related respect, such as (non-exclusive list): pH stability, temperature stability, pH profile, temperature profile, specific activity (in particular in relation to pH and temperature), substrate specificity, substrate cleavage pattern, substrate binding, position specificity, the velocity and level of release of phosphate from corn, reaction rate, phytate degradation rate), end level of released phosphate reached.

Preferred amended activity characteristics are amended specific activity, preferably increased, and preferably increased at a pH of 3, 4, 5, or 6; amended pH or temperature profile; and/or amended, preferably increased, thermostability, e.g. of an increased melting temperature as measured using DSC.

Preferred phytase variants are: Phytase variants which, when aligned according to Fig. 1, are amended as compared to a model phytase in at least one of the following positions, using the position numbering corresponding to P_lycii: 43; 44; 47; 51; 58; 62; 78; 80; 83; 88; 90; 102; 143; 148; 153; 154; 186; 187a; 195; 198; 201e; 204; 205; 211; 215; 220; 242; 244; 251e; 260; 264; 265; 267; 270; 273; 278; 302; 336; 337; 339; 352; 365; 373; 383k; 404; 417.

The following variants of A_fumigatus constitute a subgroup: Q43L; Q270L; G273D,K; N336S; A205E; Y278H; Q43L+Q270L; Q43L+Q270L+G273D; Q43L+Q270L+G273D+N336S; G273K+A205E; G273K+A205E+Y278H (see EP 0897010).

Generally, variants can be deduced or identified as follows: Looking at the alignment according to Fig. 1, comparing two sequences, one of which is a model phytase with improved properties, identifying amino acid differences in relevant positions/areas, and transferring (substituting with) from the model to the other phytase sequence the amino acid in a relevant position.

The invention also discloses a process for preparing a phytase variant which includes the above method, and further includes the deducement and synthesis of the corresponding DNA sequence, the transformation of a host cell, the cultivation of the host cell and the recovery of the phytase variant.

Relevant positions/areas include those mentioned below in relation to important phytase activity characteristics such as specific activity, thermostability, pH activity/stability.

The present invention also discloses phytase variants (varied according to a model phytase as defined herein) which are obtainable, preferably obtained, by the process outlined above and which are expected to exhibit an amended characteristic/property, preferably does exhibit such amended characteristic, e.g. an improved specific activity.

At least the basidiomycete model phytases P_lycii and T_pubescens exhibit a high specific activity (as determined using the method of Example 2 herein).

This is an example of a desired property which can be transferred to other phytases, e.g. the other phytases listed in Fig. 1, in particular to the A_pediades and the ascomycete phytases such as A_fumigatus, A-ficuum, consphyA, by a deducement process such as the one mentioned above.

Thus, amended specific activity, in particular an improved specific activity, in particular at low pH and/or high temperature, is expected from variants, which have been amended in relevant areas, viz. (i) in the amino acid residues which point into the active site cleft; or (ii) in the amino acid residues in the close neighbourhood of these active site residues. Preferably, close neighbourhood means within 10Å from the active site residues.

From the pdb file 1IHP (Brookhaven Database entry of 18.03.98 re 1IHP, Structure of Phosphomonoesterase, D.Kostrewa; or as published in Nature Structural Biology, 4, 1997, p. 185-190), active site regions can be identified, using the program INSIGHTII from Molecular Simulations MSI, San Diego, California, and using the subset command, an "active site shell" can be defined comprising those amino acid residues which lie close to the catalytic residues, defined as H59, D339 and R58 in A. ficuum phytase (corresponding to Peniophora numbers H71, D335 and R70, respectively). An "active site shell (10Å)" comprises those residues which lie within 10Å from the above catalytic residues.

The residues within 10Å from H71 and D335 are the following (using Peniophora numbers): 41-47, 68-77, 115-118, 120-126, 128, 149-163, 185, 191-193, 199, 243, 270-271, 273-275, 277-279, 288, 332-343, 364-367, 369-375, 394 ("the active site shell (10Å)").

Preferably, a "substrate binding shell" can also be defined which comprises those residues which are in close proximity to the substrate binding site and which can therefore be expected to be in contact with the substrate.

This information can be deduced as described above, by docking a sugar analogue to phytin into the active site cleft (the residues making up the surface of the active site). If a sugar without any phosphate groups is docked into the active site cleft, e.g. alpha-D-glucose (chair conformation, structure provided by the INSIGHTII program), using a fixed distance as shown below, the residues pointing towards the active site cleft can be extracted using the subset command and using a distance of 10Å from the substrate analogue. Alternatively, the compound inositol-1,4,5-triphosphate (Brookhaven database file 1djx. Inositol-1,4,5-triphosphate) can be docked into the active site cleft. This compound and glucose, however, are more or less superimposable.

The distances in Ångström (Å) are: From oxygen atom in position 6 of the alpha-D-glucose to

| | |
|---|---|
| atom ND1 of H59: | 5.84 |
| atom NH2 of R58: | 6.77 |
| atom NH2 of R142: | 5.09 |
| atom ND2 of N340: | 3.00 |
| atom ND1 of H59: | 7.76 |
| atom NH2 of R58: | 8.58. |

(the Peniophora numbers of the above residues are: H71, R70, R155, N336, H71 and R70, respectively).

In this way, the residues in contact with the substrate are identified as follows (Peniophora numbers): 43-44; 70-80; 83-84; 115; 153; 155-156; 184; 191-192; 198-202; 205; 235; 238; 242; 270; 272-273; 275-277; 332-336; 338; 369; 371 ("the substrate binding shell (10Å)").

Variants being amended in one or more of (1) the active site shell or (2) the substrate binding shell, are strongly expected to have an amended specific activity. This leads to the following joint grouping of positions (still Peniophora numbers and 10Å shells): 41-47, 68-80, 83-84, 115-118, 120-126, 128, 149-163, 184-185, 191-193, 198-201e, 202-203, 205, 235-236, 238-239, 242-243, 270-279, 285, 288, 332-343, 364-367, 369-375, 394.

Preferably, the active site shell and the substrate binding shell are defined as described above using the basidiomycete model phytases of Fig. 1, the Peniophora phytase being a preferred model. A deducement of corresponding variants of other model phytases is possible using the alignment of Fig.

Preferably, a distance of 5Å is used in the subset command, thus defining active site and substrate binding shells of a more limited size, e.g. an active site shell comprising the residues 43-44, 69-74, 117, 125, 155-156, 159, 274, 332-340, 370-374 (5Å from H71 and D335), "active site shell (5Å)".

Generally the active site shell and substrate binding shell regions form the basis for selecting random mutagenesis regions. Examples of preferred random mutagenesis regions are
regions 69-74, 332-340, 370-374, doping to be added (a 5Å approach); and
regions 57-62, 142-146, 337-343, doping to be added (a 10Å approach).

It is presently contemplated that any amendment in either of these positions will lead to a phytase of amended characteristics, e.g. of an amended specific activity.

The above expression "any amendment in either of the positions" is considered fully equivalent to listing each position and each substitution, e.g. as follows for the above sub-group 41-47:
41A, C, D, E, F, G, H, I, K, L, M, N, P, Q, R, S, T, V, W, Y;
42A, C, D, E, F, G, H, I, K, L, M, N, P, Q, R, S, T, V, W, Y;
43A, C, D, E, F, G, H, I, K, L, M, N, P, Q, R, S, T, V, W, Y;
44A, C, D, E, F, G, H, I, K, L, M, N, P, Q, R, S, T, V, W, Y;
45A, C, D, E, F, G, H, I, K, L, M, N, P, Q, R, S, T, V, W, Y;
46A, C, D, E, F, G, H, I, K, L, M, N, P, Q, R, S, T, V, W, Y;
47A, C, D, E, F, G, H, I, K, L, M, N, P, Q, R, S, T, V, W, Y.

Amended specific activity is in particular expected from the following variants:
42S,G; 43A, C, D, E, F, G, H, I, K, L, M, N, P, Q, R, S, T, V, W, Y; 45D,S; 47Y,F; 51E,A; 75W,F; 785, D; 79G; 80K, A; 83I, Q; 84Q,V; 116S; 118V,L; 119E; 120L; 122A; 123N,T; 125S; 126H,S; 127Q,E; 128A, T; 151A, S; 152G; 153D,Y; 154Q,D,G; 157V; 158D, A; 159T; 160A, S; 161T,N; 162N; 163W; 184Q,S; 186A,E; 198A,N; 200G,V; 201D; deletions of one or more of 201a, 201b, 201c, 201d, 201e, 201f - preferably all; 202S; 205Q,E; 235Y,L; 238L,M; 242P; 270Y,A,L; 271D; 273D,K; 275F,Y; 278T,H; 332F; 336S; 337T,Q; 339V; 340P, A; 343A, S; 364W,F; 365V,L; 366D,V; 367K; 368K; 369I, L; 370V; 373S; 374A; 375H; 376M; 393V.

Particularly preferred variants are the following: 78S; 79G; 80A; 831,Q; 84Q,V; 198A,N; 200G,V; 201D; deletions in one or more of 201a, 201b, 201c, 201d, 201e, 201f - preferably all deletions; 202S; 205Q, E; 235Y, L; 238L,M; 242P, 273D; 275F, Y.

Other particularly preferred variants are the following: 43A, C, D, E, F, G, H, I, K, L, M, N, P, Q, R, S, T, V, W, Y; in particular 43M,P; 75W, F; 80K; 153D; 184Q,S; 270Y, A; 332F; 369I, L.

The following variants are especially preferred: 43L,G,N,V,A,I,T; 78D; 153Y; 154G; 270L; 273D,K. Double and triple variants (43L/270L); (43L/270L/273D); (43L/78D) and (43L/153Y/154G) are also especially preferred. Other preferred variants are 205E; 278H; 336S.

These especially preferred single, double and triple variants are preferably variants of model phytases which can be aligned to Fig.1, in particular variants of the specific model phytases listed in Fig. 1.

At least consphyA is known to have a high thermostability. Still further, the thermostability of P_lycii is rather high.

This is an example of a desired property which can be transferred to other phytases, e.g. the other phytases listed in Fig. 1, in particular to the basidiomycete phytases such as P_lycii and A_pediades, by a deducement process such as the one mentioned above.

Amended thermostability, in particular improved thermostability, is expected on this background from the following variants:
39H,S; 40L,N; 43P; 47Y,F; 49P; 51E,A; 56P; 58D; 61R; 62V; 80K; 83A; 84Y; 172P; 184P; 195T; 198A; 204V; 211L; 223D; 236Y; 242P; 246V; 253P; 264R; 265Q; 280A, P; 283P; 287A; 292F,Y; 293A; 302R; 304P; 337S; 348Y; 387P; 396R; 409R; 411K; 412R; 417E; 421F,Y.

The following variants of amended thermostability are particularly preferred: 39S; 40N; 47Y,F; 51A; 83A; 195T; 204V; 211L; 242P; 265A.

Further variants of amended thermostability are the following: 42G; 43T,L,G; 44N; 58K,A; 59G; 62I; 69Q; 75F; 78D; 79G; 80A; 81A, G; 82T; 83K,R; 84I; 88I; 90R, A; 102Y; 115N; 118V; 122A; 123Q,N; 125M,S; 126V,S; 127N,Q; 128S, A; 143N,K; 148V, I; 154S; 158D; 170fH; 170gA; 171T,N; 172N; 173W, 184S; 186A; 187A; 187aS; 193S; 195V,L; 198V; 201E; 201eT; 202A, 203aT; 204A; 211V, 215P, A; 220L,N; 223H; 228N; 232T; 322E; 235T; 236N; 242S; 244D; 251eQ, E; 256D; 264I; 260A,H; 265A; 267D; 270G; 271D; 273K,D; 278T,H; 287T; 293V; 302H; 337T,G; 338I; 339V,I; 340A; 352K; 365A, S; 366S; 367A; 369L; 373S, A; 374S; 376M; 383kE, Q; 404G, A; 411T; 417R; 431E.

Other concepts, which can be expected to impart an improved thermostability to a phytase, are as follows - considering the 1IHP structure previously referred to and transferring via an alignment according to Fig. 1 as outlined herein:
(A) Introduction of prolin residues in spatial positions where the prolin special dihedral angles are satisfied and the hydrogen bonding network are not hampered and no steric clashes are observed.
(B) Filling up holes: By substitution for bigger residues in internal cavities an improvement in stability can often be obtained.
(C) Cystin bridge: Cystin bridges will often make the proteins more rigid and increase the energy of unfolding.

Further variants from which amended thermostability is expected according to these concepts of (A) to (C) are: 27P, 31Y, 132F, 132I, 132L, 184P, 186P, 190P, 280P, 343F, 343I, 343L, 349P, 362P and (33C and 24C).
Concept (A): 27P, 184P, 186P, 190P, 349P, 362P.
Concept (B): 343F, I, L; 31Y; 132F,I,L; 273F.
Concept (C): 33C/24C.

Amended pH activity or stability, preferably stability, in particular at low pH, in particular improved, is another desired property which can be transferred by aligning according to Fig. 1 and transferring from models of improved pH profiles to other phytases - as outlined above.

Other concepts, which can be expected to impart an improved stability at low pH to a phytase, are as follows - considering the 1IHP structure previously referred to and transferring via an alignment according to Fig. 1 as outlined herein:
(D) Surface charges: Better distribution at low pH, to avoid cluster of negative or positive, and to avoid too close same charged residues.
(E) Prevent deamidation: Surface exposed Q or N in close contact to negative charged residues.

Phytase variants having improved pH stability/activity at low pH are expected to be: 39H; 39Q; 80A; 203R; 271N; 51R; 154S; 185S; 194S; 194T; 288L; 288I; 288F; 360R; 173Q,S; 204Q,S; 303K,S; 81Q,E.
Concept (D): 203R, 271N, 51R, 185S, 360R; 173Q, S; 204Q,S; 303K,S; 81Q, E.
Concept (E): 154S; 194S, T; 288L, I, F.

A preferred model phytase for these concepts of (D) and (E) is P_lycii.

Experimentally proven to have a lowered pH optimum is: Variant 80A of ascomycete phytases, in particular of A_fumigatus and consphyA.

Especially preferred single, double and triple variants are 43L; (43L/270L) and (43L/270L/273D). These variants have a changed pH profile. They are preferably variants of the specific model phytases listed in Fig. 1.

For all preferred variants listed above:
the stability is preferably amended at high temperature, viz. in the temperature range of 50-100°C, in particular 60-90°C, more preferably in the range of 70-90°C;
the activity is preferably amended in a temperature range relevant for the use in the gastro-intestinal system of animals, e.g. 30-40°C, more preferably 32-38°C, most preferably in the range of 35-38°C;
the stability is preferably amended at low pH, viz. in the pH range of pH 1.5-7, preferably 2-6, more preferably 3-5;
the activity is preferably amended in the pH range of pH 1.5-5.5, more preferably at pH 2.5-4.5, still more preferably 3-5.

Tests for amended phytase characteristics, such as those mentioned above, are well known in the art and any such test can be used to compare the performance of the phytase variants with the phytase models.

A preferred test for specific activity is given in Example 2. Preferred tests for pH and temperature activity and stability are given in Example 3. An even more preferred test for thermal stability is the DSC method of Example 4.

WO 98/28409 discloses tests for various other parameters, too, such as position specificity. All the tests of WO 98/28409 are preferred tests.

Generally, of course all these tests can be conducted at desired pH values and temperatures.

In an analogous way other preferred variants based on the remaining eight specifically disclosed model phytases can easily be deduced by combining the suggested amendments with each of the corresponding sequences of Fig. 1. These preferred variants are easily deduced, viz. the following:

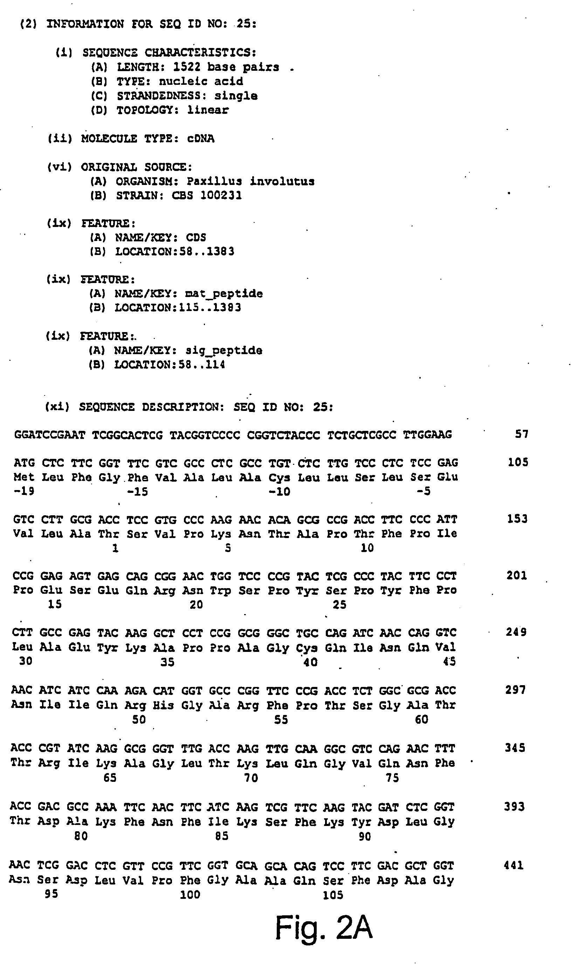

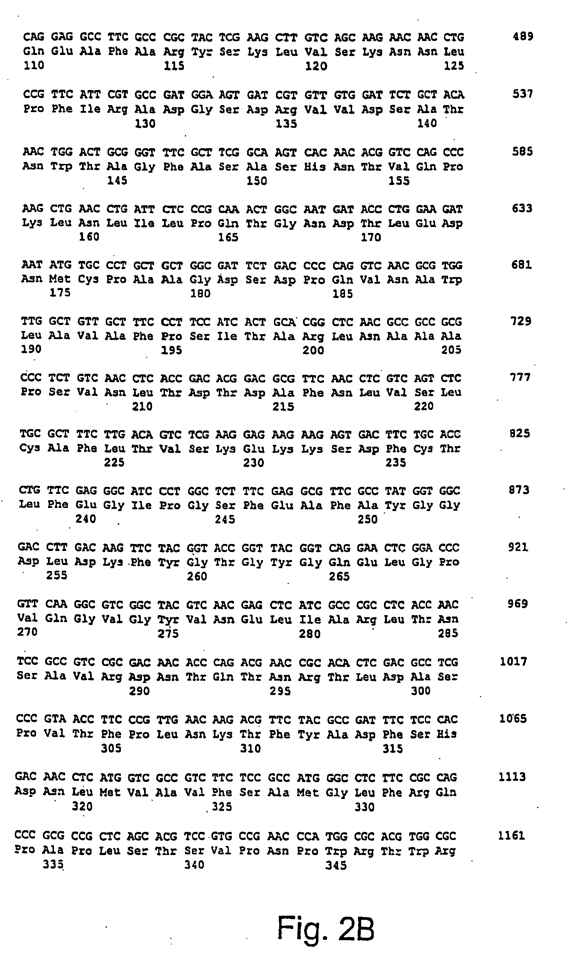

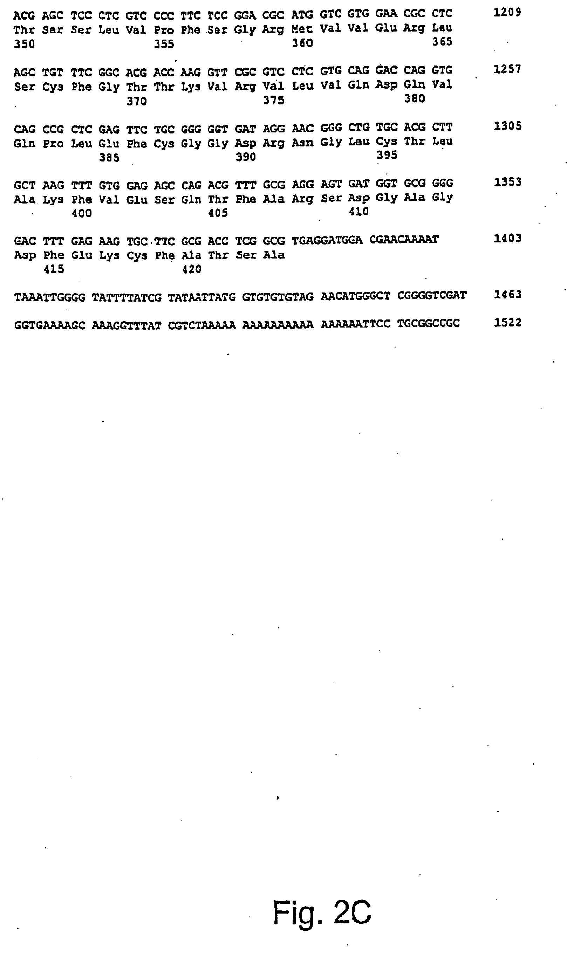

Variants of a model phytase derived from Paxillus, preferably Paxillus involutus, preferably derived from strain CBS 100231, preferably variants of P_involtus-A1, the sequence of which is shown at Fig. 2, said variants comprising at least one of the following amendments:
() 24C; T27P; F31Y; I33C; R39H,S,Q; N40L; S42G;
   P43A, C, D, E, F, G, H, I, K, L, M, N, Q, R, S, T, V, W, Y; Y44N; S45D; Y47F; A51E, R; A58D; K; Q61R; 162V; F75W; S78D; A80K; T81Q, E, G, A; R83A, I, Q, K; I84Y, Q, V; L88I; K90R, A; F102Y; S115N; D116S; V118L; P119E; F120L; A123N,T,Q; S125M; F126H,S,V; D127Q,E,N; A128T,S; A132F, I, L; I148V; D151A, S; S153D, Y; D154Q,S,G; D158A; S159T; A160S; T161N; ()170fH; () 170gA; S171N; H172P; N173Q, S; P184Q, S; Q185S; T186A,E,P; G187A; ()187aS; T190P,A; D193S; N194S,T; M195T,V,L; A198N,V; G200V; D201E; ()201eT; S202A; D203R,K,S; P203aV,T; Q204E, S, A, V; V205E; V211L; S215A, I; L220N; A223D, H; D233E; F235Y,L,T; N236Y; L237F; V238L,M; A242P,S; M244D; () 251eE,Q; D253P; T256D; P260A,H; E264R, I; A265Q; A267D; G270Y, A, L; D271N; D273K; F275Y; T278H; Y280A,P; E283P; V287A, T; Q288L, I, F; Y292F; V293A; N302R,H; A304P; N336S; L337T,Q,S,G; M 338I; V339I; A340P; S343A, F, I, L; F348Y; R349P; A352K; P360R; R362P; W364F; R365V, L, A, S; T366D,V,S; S367K, A; S368K; L369I; S373A; G374A,S; R375H; ()383kQ,E; T387P; Q396R; G404A; L409R; T411K; L412R; E417R; F421Y.

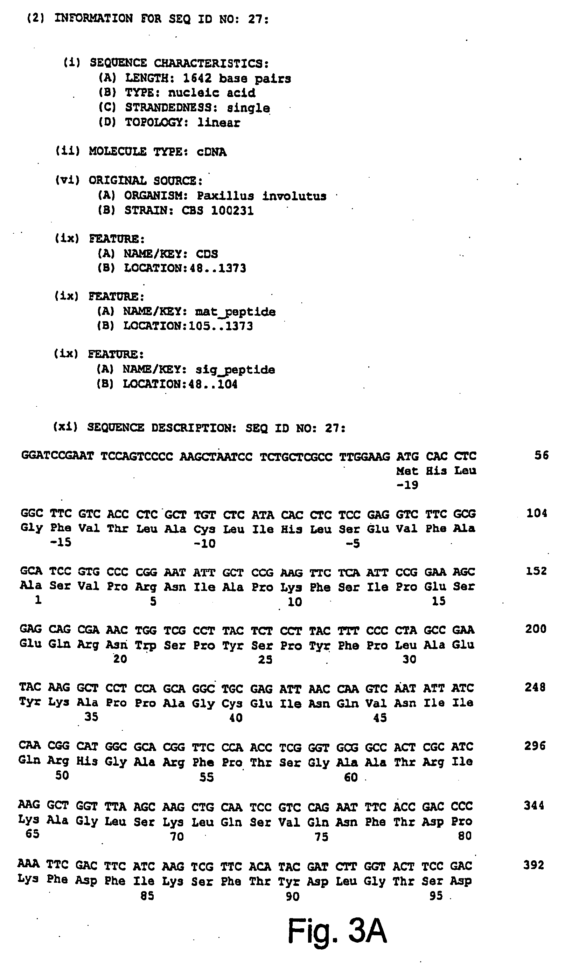

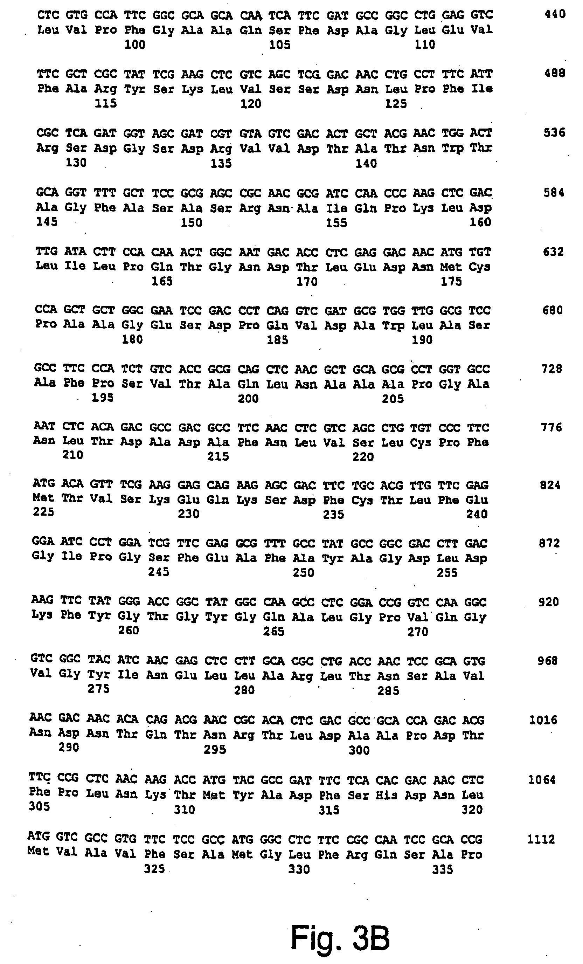

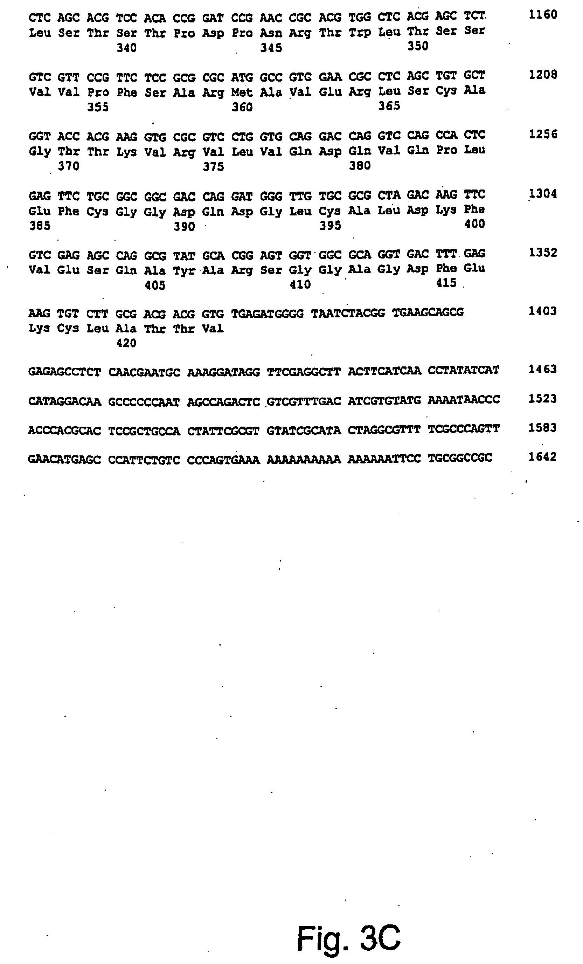

Variants of a model phytase derived from a species of the genus Paxillus, preferably the species Paxillus involutus, preferably derived from strain CBS 100231, preferably variants of P_involtus-A2, the sequence of which is shown at Fig. 3, said variants comprising at least one of the following amendments:
P24C; I27P; F31Y; I33C; R39H,S,Q; N40L; S42G;
   P43A, C, D, E, F, G, H, I, K, L, M, N, Q, R, S, T, V, W, Y; Y44N; S45D; Y47F; A51E,R; A58D, K; E61R; 162V; F75W; S78D; A80K; A81Q, E, G; R83A, I, Q, R, K; I84Y, Q, V; L88I; K90R,A; F102Y; S115N; D116S; V118L; P119E; F120L; A123N,T,Q; S125M; F126H,S,V; D127Q,E,N; A128T,S; V132F, I, L; D143N; I148V; D151A, S; S153D,Y; D154Q, S, G; D158A; A160S; T161N; ()170fH; ()170gA; S171N; R172P; N173Q,S; P184Q,S; Q185S; T186A, E, P; G187A; ()187aS; T190P,A; D193S; N194S,T; M195T,V,L; A198N,V; G200V; E201D; ()201eT; S202A; D203R,K,S; P203aV,T; Q204E,S,A,V; V205E; S211L,V; S215A, P; L220N; A223D,H; A232T; F235Y, L, T; N236Y; L237F; V238L,M; P242S; M244D; () 251eE, Q; D253P; T256D; P260A,H; E264R, I; A265Q; A267D; G270Y, A, L; D271N; D273K; F275Y; T278H; Y280A,P; A283P; V287A, T; Q288L, I, F; Y292F; I293A, V; N302R,H; A304P; N336S; L337T, Q, S, G; M338I; V339I; 340P,A; A343S,F,I,L; F348Y; R349P; A352K; P360R; R362P; W364F; L365V, A, S; T366D,V,S; S367K, A; S368K; V369I, L; S373A; R375H; ()383kQ,E; T387P; Q396R; G404A; L409R; A411K,T; L412R; E417R; Y421F.

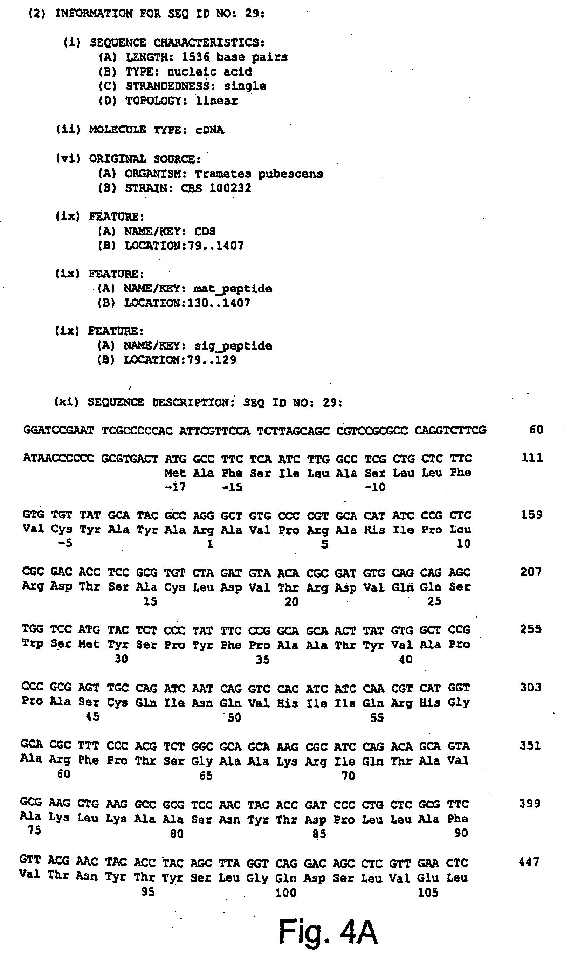

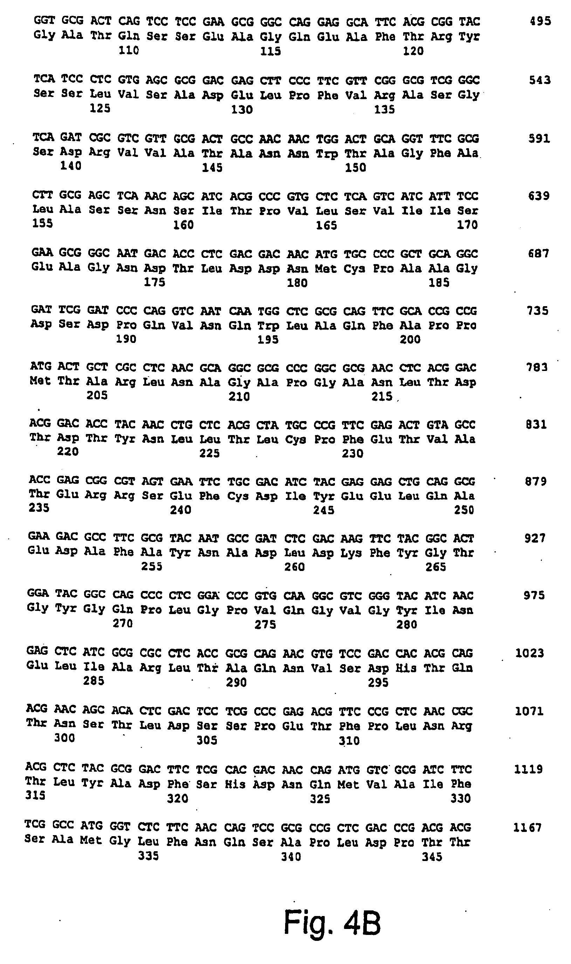

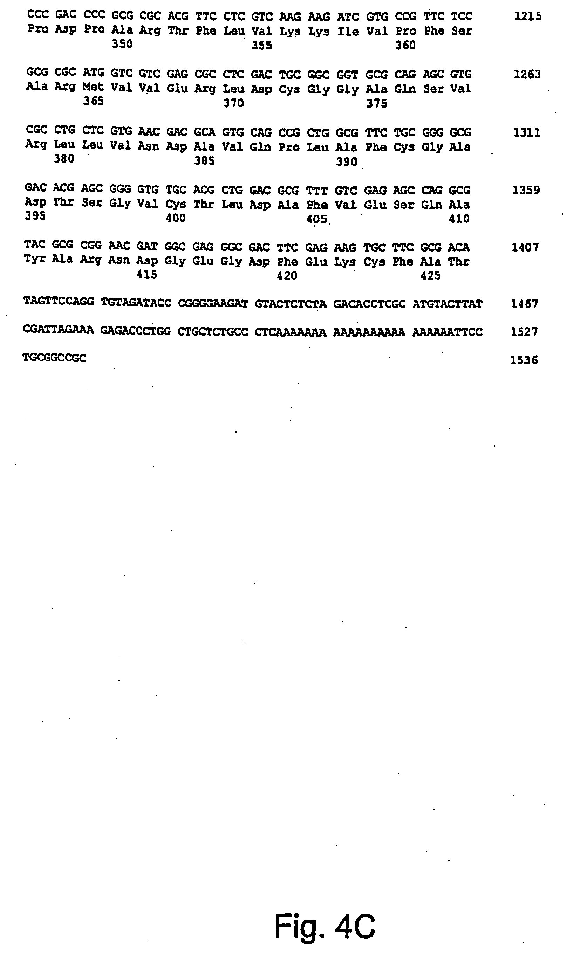

Variants of a model phytase derived from a species of the genus Trametes, preferably the species Trametes pubescens, preferably derived from strain CBS 100232, preferably variants of T_pubescens, the sequence of which is shown at Fig. 4, said variants comprising at least one of the following amendments:
R24C; T27P; L31Y; V33C; Q39H,S; S40L,N; S42G;
   M43A, C, D, E, F, G, H, I, K, L, N, P, Q, R, S, T, V, W, Y; Y44N; S45D; Y47F; A51E,R; A58D,K; S59G; Q61R; 162V; F75W; S78D; A80K; A81Q, E, G; R83A, I, Q, K; I84Y, Q, V; V88I; K90R,A; L102Y; D115N; V118L; T123N,Q; S125M; S126H,V; E127Q,N; A128T,S; A132F, I, L; D143N; V148I; S151A; S153D,Y; D154Q,S,G; A158D; A160S; N161T; ()170fH; ()170gA; S171N; S172P; N173Q,S; S184Q,P; E185S; A186E,P; G187A; () 187aS; T190P,A; N194S,T; M195T,V,L; A198N,V; G200V; () 201eT; S202A; D203R, K, S; P203aV,T; Q204E,S,A,V; V205E; Q211L,V; P215A; L220N; G223D,H; D233E; Y235L,T; N236Y; L237F; L238M; P242S; E244D; () 251eE, Q; E253P; Q260A, H; D264R, I; A265Q; A267D; A270Y,L,G; D271N; D273K; F275Y; T278H; Y280A,P; V287A,T; Q288L, I, F; Y292F; I293A, V; A302R,H; N304P,A; N336S; Q337T, S, G; M338I; V339I; A340P; S343A, F, I, L; F348Y; N349P; A352K; P360R; R362P; F364W; L365V,A,S; V366D,S; K367A; I369L; A373S; A374S; R375H; ()383kQ,E; Q387P; A396R; G404A; V409R; T411K; L412R; E417R; Y421F.

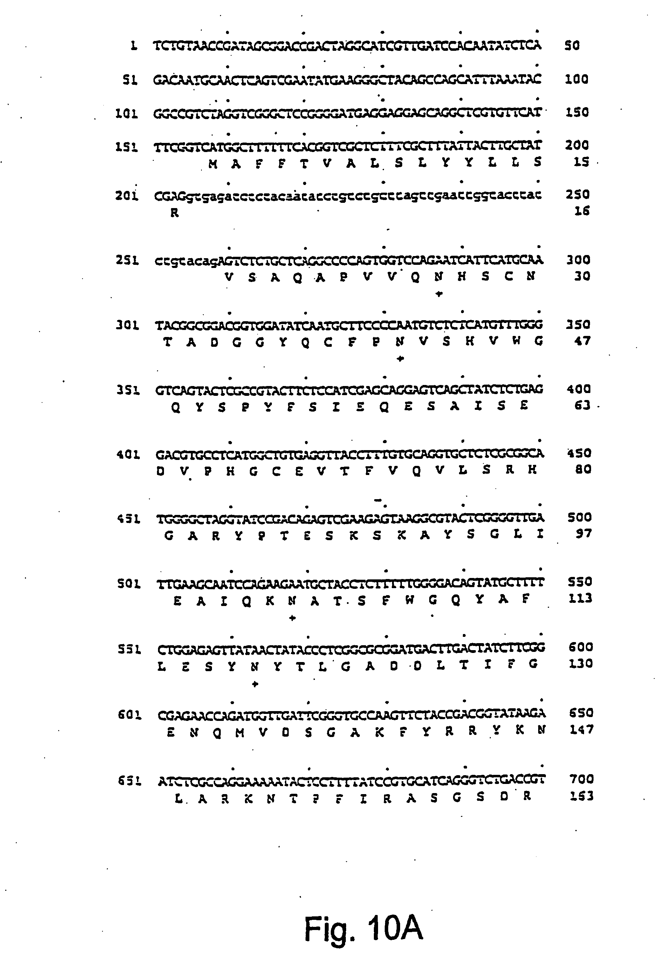

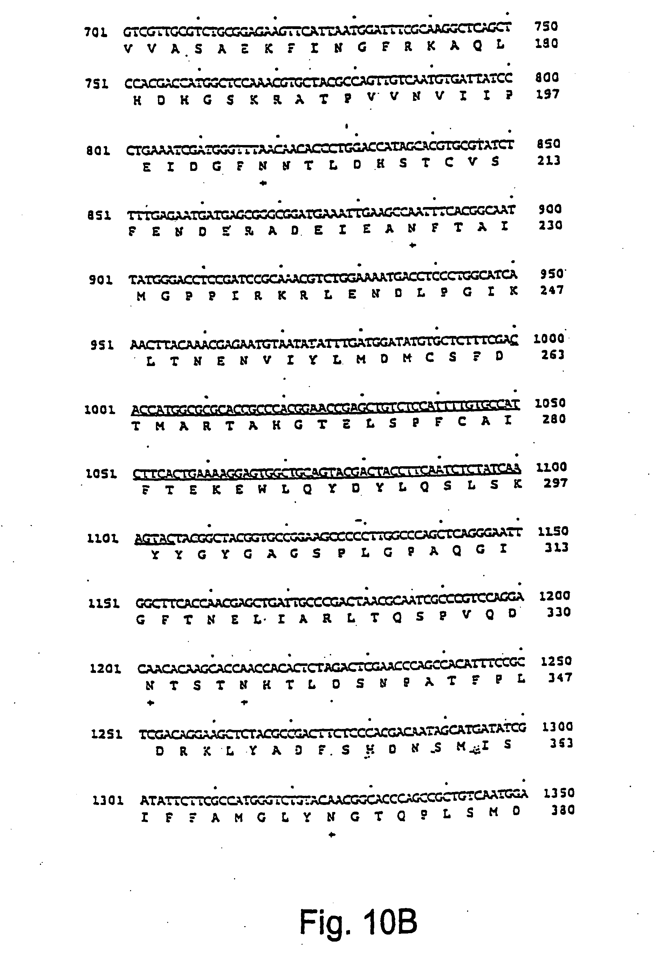

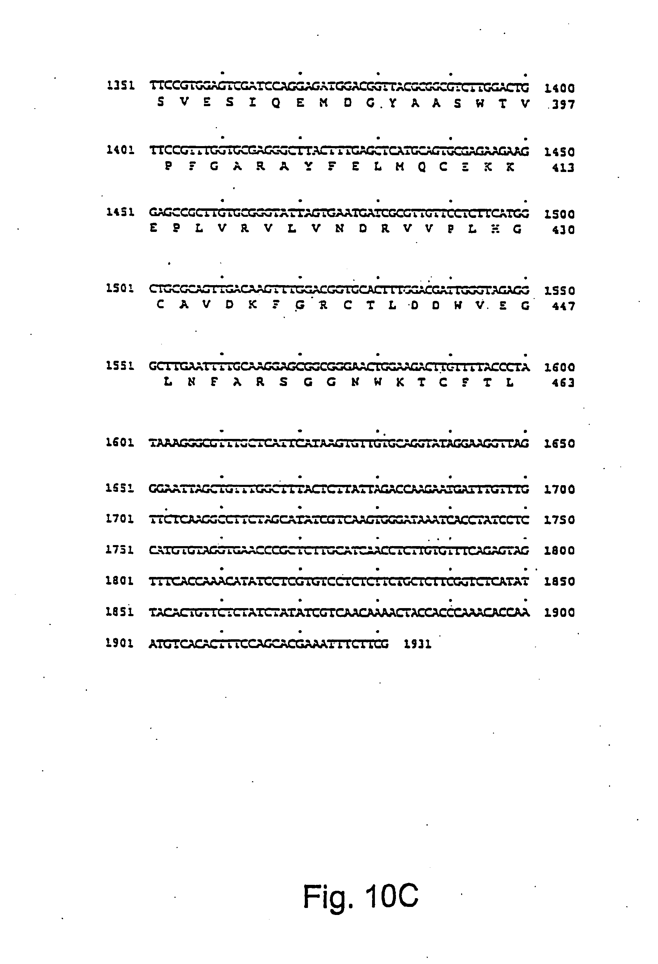

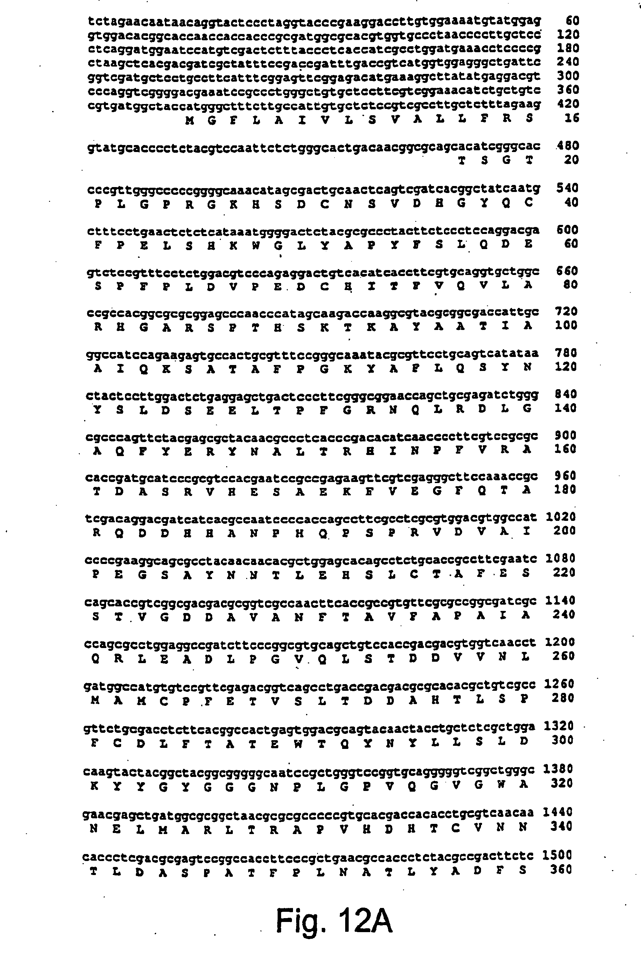

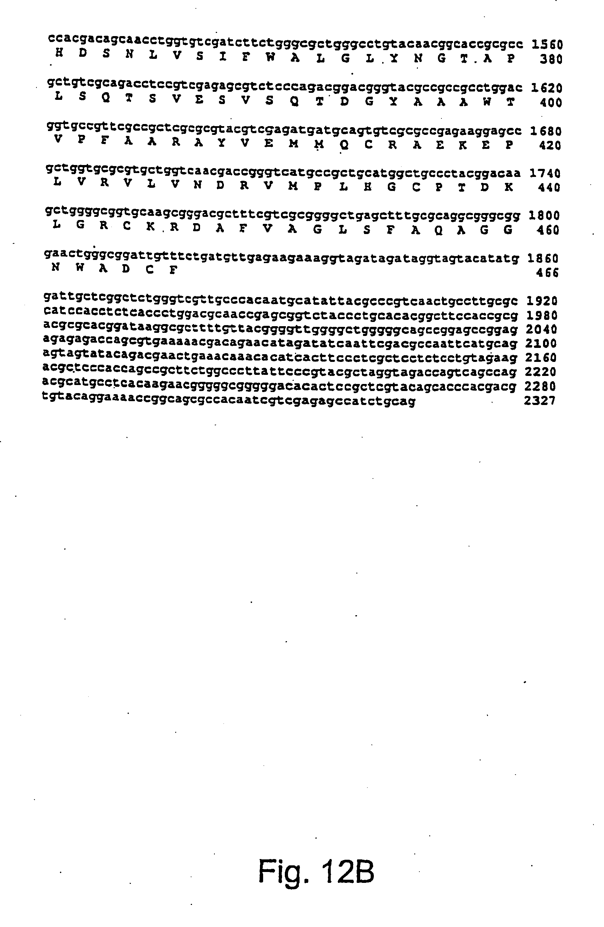

Variants of a model phytase derived from a species of the genus Aspergillus, preferably the species Aspergillus nidulans, preferably derived from strain DSM 9743, preferably variants of A_nidulans, the sequence of which is shown at Fig. 10, said variants comprising at least one of the following amendments:
V24C; A27P; H39S,Q; V40L,N; G42S;
   Q43A, C, D, E, F, G, H, I, K, L, M, N, P, R, S, T, V, W, Y; Y44N; S45D; Y47F; S49P; E51A,R; V56P; H58D,K,A; E61R; V62I; S69Q; Y75W, F; E78D,S; S79G; K80A; S81Q,E,A,G; K82T; A83I,Q,K,R; Y84Q,V,I; A90R; D115N; D116S; T118V,L; I119E; F120L; E122A; N123T,Q; M125S; V126H,S; D127Q,E,N; S128A,T; F132I,L; K143N; I148V; S151A; S153D,Y; D154Q,S,G; A158D; S159T; A160S; E161T,N; K162N; F163W; G170fH; S170gA; ()171N; ()172P; K173Q,S; P184Q,S; E185S; I186A,E,P; D187A; G187aS; T190P,A; H193S; S194T; S198A,N,V; E200G,V; N201D,E; D201e(); E201e (), T; R201f() (a deletion of at least one of 201d, 201e, 201f, preferably all); A202S; D203R,K,S; E203aV,T; I204Q, E, S, A, V; I211L,V; P215A; L220N; D223H; K228N; E232T; N233E; I235Y,L,T; Y236N; L237F; M238L; S242P; M246V; E251eQ; A256D; E260A,H; L264R,I; Q270Y, A, L, G; S271D,N; S273D,K; Y275F; G278T,H; A280P; A287T; Q288L, I, F; F292Y; T293A,V; Q302R,H; P304A; N336S; S337T,Q,G; M338I; I339V; S340P,A; F343A, S, I, L; N349P; Q352K; S360R; Q362P; Y364W, F; A365V,L,S; A366D, V, S; S367K, A; W368K; T369I, L; G373S, A; A374S; R375H; A376M; E383kQ; A404G; T411K; L412R; E417R; F421Y; K431E.
Variants of a model phytase derived from a species of Aspergillus, preferably Aspergillus terreus, preferably derived from strain CBS 220.95, preferably variants of A_terreus, the sequence of which is shown at Fig. 12, said variants comprising at least one of the following amendments:
   G24C; V27P; H39S,Q; K40L,N; G42S;
      L43A, C, D, E, F, G, H, I, K, M, N, P, Q, R, S, T, V, W, Y; Y44N; A45D, S; Y47F; S49P; Q51E, A, R; V56P; P58D,K,A; D59G; H61R; I62V; A69Q; S75W, F; H78D, S: S79G; K80A; T81Q, E, A, G; A83I, Q, K, R; Y84Q, V, I; A90R; E115N; E116S; T118V,L; P119E; F120L; R122A; N123T,Q; L125S,H; R126H,S,V; D127Q,E,N; L128A,T,S; F132I,L; H143N; V148I; T151A,S; D152G; A153D, Y; S154D,Q,G; H157V; E158D, A; S159T; A160S; E161T,N; K162N; F163W; H173Q,S; P184Q,S; E185S; G186A,E,P; S187A; A187aS; T190P, A; H193S; S194T; L195T, V; A198N,V; E200G,V; S201D,E; S201d () ; T201e () ; V201f () ; G202S,A; D203R,K,S; D203aV,T; A204Q,E,S,V; V205E; V211L; A215P; L220N; D223H; Q228N; D232T; D233E; V235Y,L,T; N236Y; L237F; M238L; P242S; E244E; T251eE,Q; A260H; T264R,I; Q265A; N267D; L270Y,A,G; S271D,N; K273D; Y275F; H278T; G280A,P; V287A,T; Q288L,I,F; W292F,Y; A293V; Q302H; P304A; N337T,Q,S,G; L338I; V339I; S340P,A; W343A,S,F,I,L; N349P; A352K; S360R; S362P; Y364W,F; A365V,L,S; A366D,V,S; A367K; W368K; T369I,L; A373S; A374S; R375H; A376M; R383kQ,E; P404A,G; K411T; A417E, R; F421Y;. A431E.

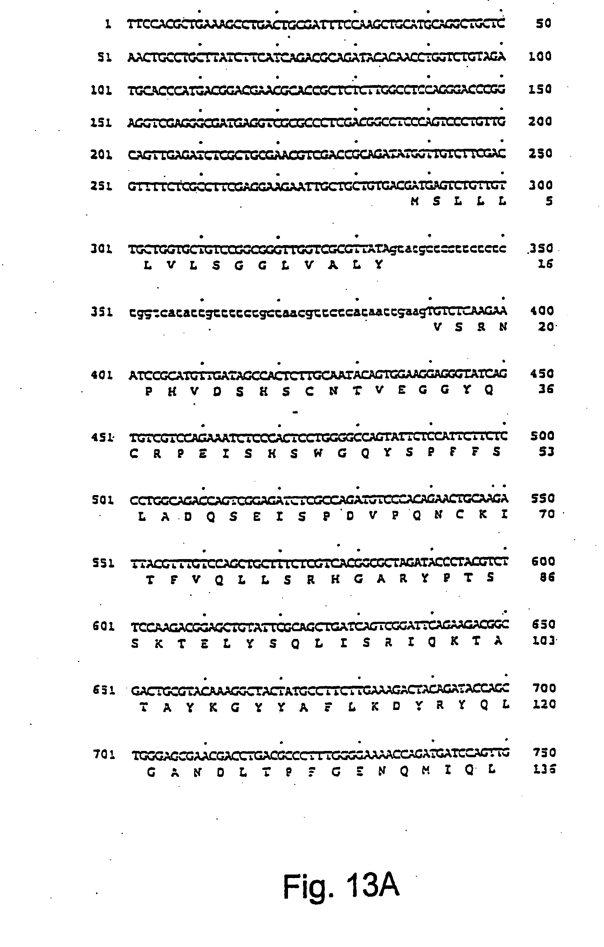

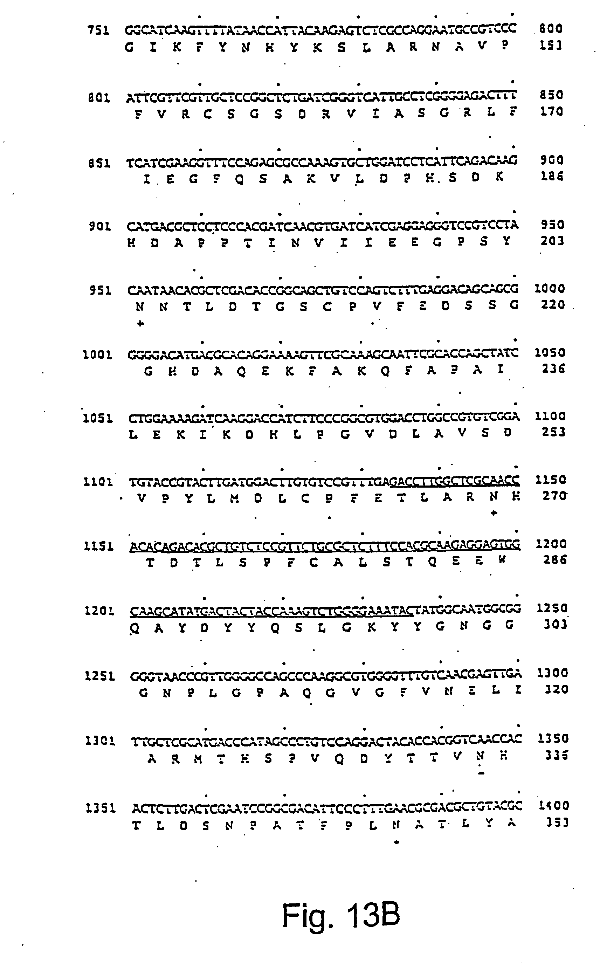

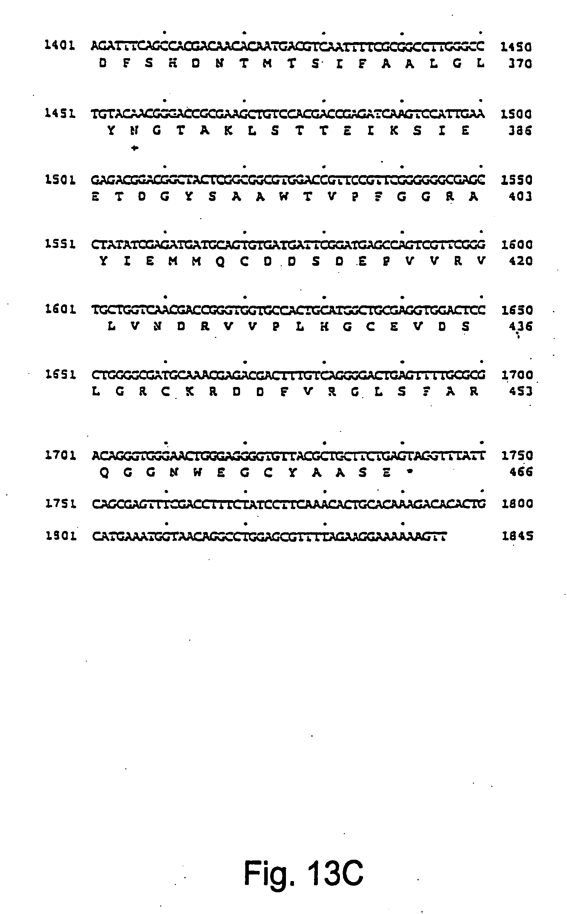

Variants of a model phytase derived from a species of Talaromyces, preferably the species Talaromyces thermophilus, preferably derived from strain ATCC 20186 or ATCC 74338, preferably variants of T_thermo, the sequence of which is shown at Fig. 13, said variants comprising at least one of the following amendments:
H24C; V27P; H39S,Q; S40L,N; G42S;
   Q43A, C, D, E, F, G, H, I, K, L, M, N, P, R, S, T, V, W, Y; Y44N; S45D; F47Y; S49P; A51E, R; V56P; Q58D, K, A; N59G; K61R; I62V; Y75W, F; S78D; S79G; K80A; T81Q, E, A, G; E82T; L83A, I, Q, R, K; Y84Q, V, I: R90A; D116S; T118V,L; P119E; F120L; E122A; N123T,Q; M125S: I126H,S,V; Q127E,N; L128A,T,S; F132I,L; V148I; S151A; S153D,Y; D154Q,S,G; I157V; A158D; S159T; G160A,S; R161T,N; L162N; F163W; S170gA; D171N; K172P; H173Q,S; E184Q,S,P; E185S; G186A,E,P: D187A; T190P,A; T193S; G194S,T; S195T,V,L; V198A,N; E200G,V; D201E; S201d () ; S201e () , T; S201f () ; G202S, A; H203R, K, S; D203aV,T; A204Q,E,S,V; Q205E; Q211L,V; A215P; I220N,L; H223D; D228N; S232T; D233E; P235Y,L,T; Y236N; M237F; D238L,M; P242S; E244D; L246V; () 251eE, Q; A256D; Q260A, H; Q264R, I; A265Q; Q270Y, A, L, G; S271D,N; G273D,K; Y275F; N278T,H; G280A,P; A287T; Q288L, I, F; F292Y; V293A; H302R; P304A; N336S; T337Q, S, G; M338I; T339V, I; S340P, A; A343S, F, I, L; N349P; A352K; S360R; E362P; Y364W,F; S365V, L, A; A366D,V,S; A367K; W368K; T369I, L; G373S, A; G374A, S; R375H; A376M; D383kQ,E; E404A; K411T; R417E; F421Y.

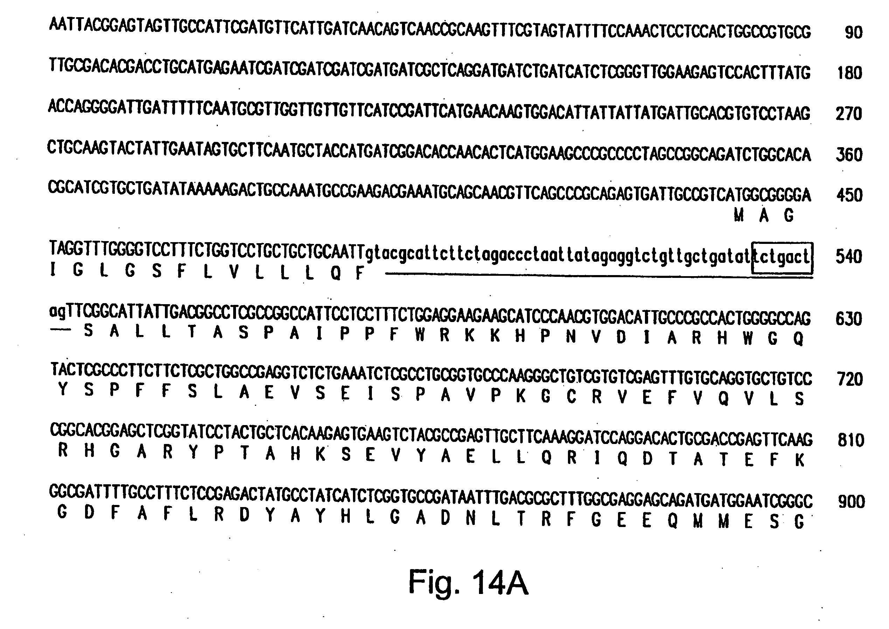

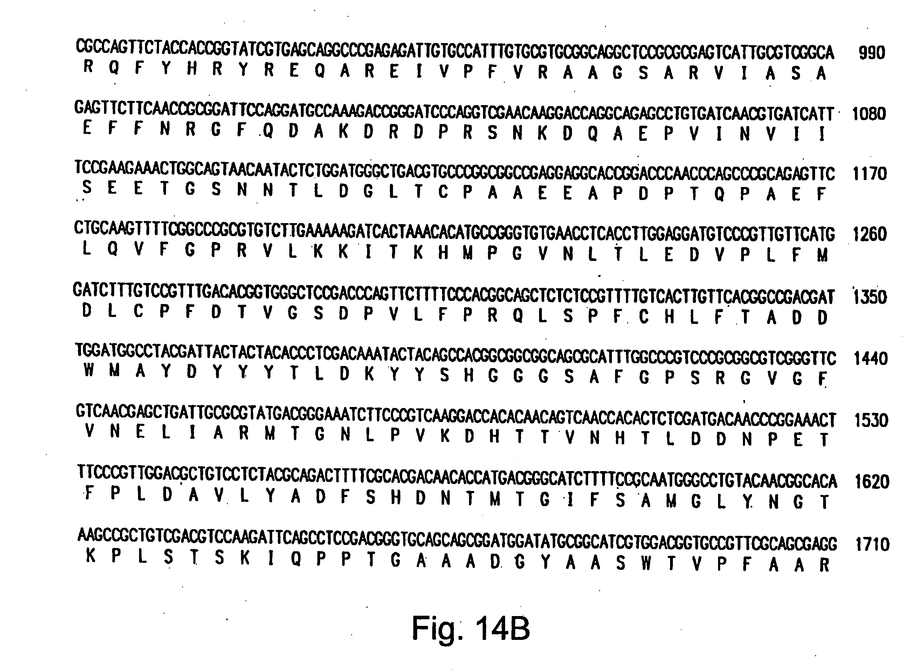

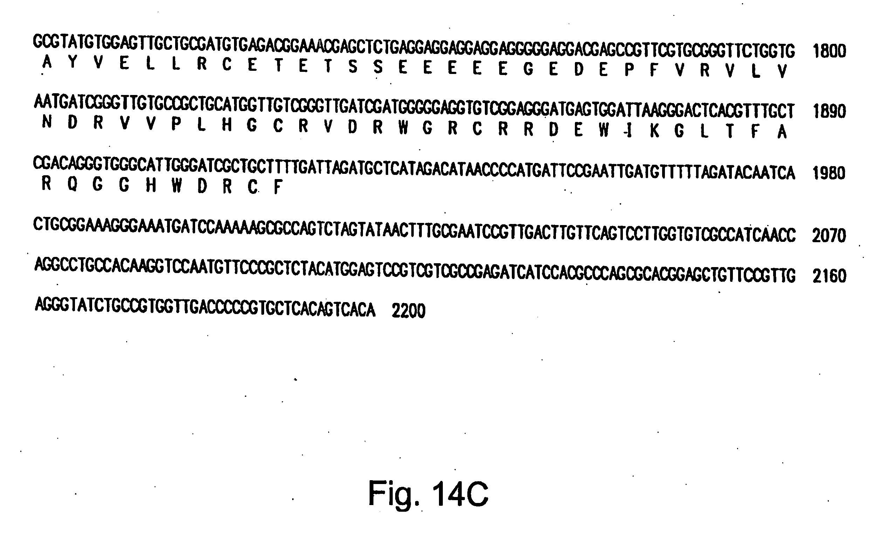

Variants of a model phytase derived from a species of Thermomyces, preferably the species Thermomyces lanuginosus, preferably derived from strain DBS 586.94, preferably variants of T_lanuginosa, the sequence of which is shown at Fig. 14, said variants comprising at least one of the following amendments:
K24C; ()27P; ()31Y; ()33C; R39H,S,Q; H40L,N; G42S;
   Q43A, C, D, E, F, G, H; I, K, L, M, N, P, R, S, T, V, W, Y; Y44N; S45D; F47Y; S49P; A51E,R; V56P; K58D,A; V62I; S69Q; Y75W,F; A78D,S; H79G; K80A; S81Q,E,A,G; E82T; V83A,I,Q,K,R; Y84Q,V,I; L88I; R90A; F102Y; D115N; N116S; T118V,L; R119E; F120L; E122A; E123N,T,Q; M125S; M126H,S,V; E127Q,N; S128A,T; F132I,L; E143N; V148I; A151S; S153D,Y; A154D,Q,S,G; I157V; A158D; S159T; A160S; E161T,N; F162N; F163W; R170fH; S170gA; K172P; D173Q,S; S184Q,P; E185S; E186A,P; T187A; G187aS; T190P,A; G193S; L194S,T; T195V,L; A198N,V; E200G,V; E201D; A201d () ; P201e () , T; D202S, A; P203R,K,S; T203aV; Q204E, S, A, V; P205E; V211L; R215A,P; I220L, N; H223D; E232T; D233E; P235Y,L,T; L236Y,N; M238L; P242S; Q251eE; H256D; Q260H; M264R, I; A265Q; Y270A,L,G; T271D,N; D273K; Y275F; H278T; G280A,P; A283P; S287A; R288L, I, F; F292Y; V293A; G302R,H; P304A; N336S; T337Q,S,G; M338I; T339V, I; G340P,A; S343A, F, I, L; N349P; P360R; T362P; Y364W,F; A365V, L, S; A366D, V, S; S367K, A; W368K; T369I,L; A373S; A374S; R375H; A376M; E383kQ; R404A,G; R411K,T; K417E,R; F421Y; D431E.

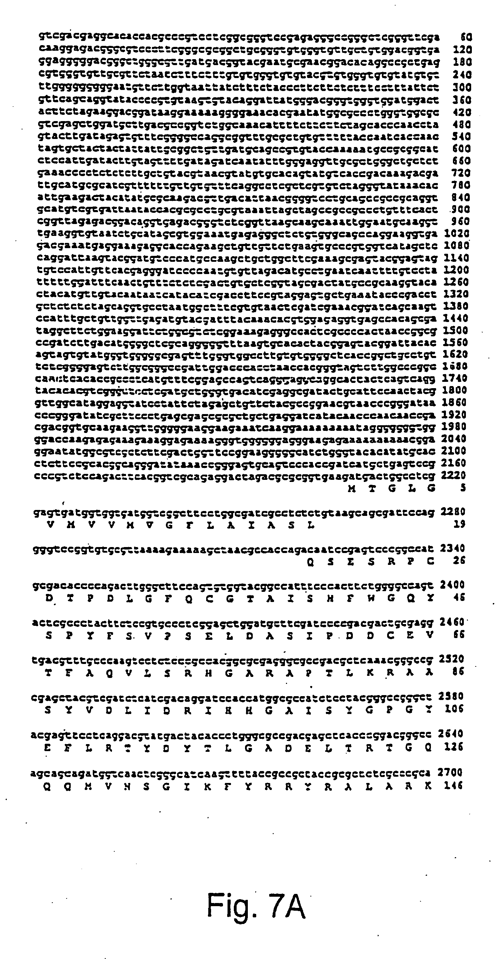

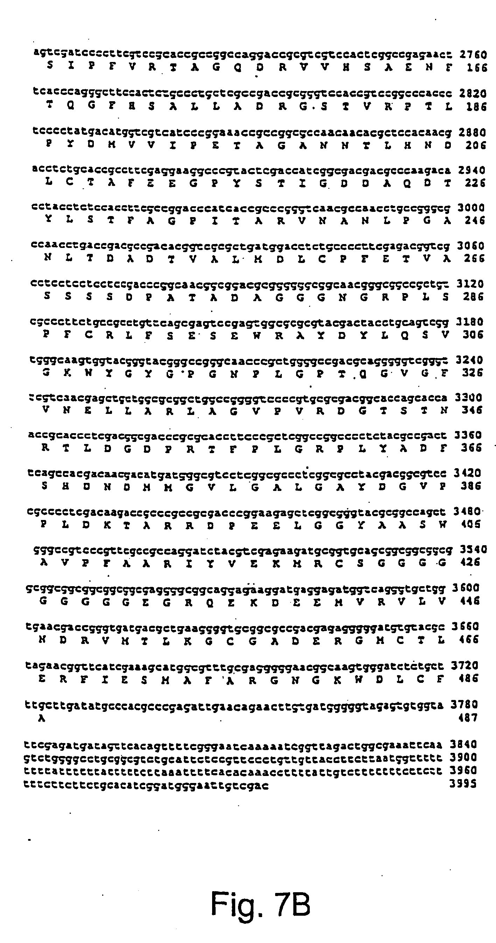

Variants of a model phytase derived from a species of Myceliophthora, preferably the species Myceliophthora thermophila, preferably derived from strain ATCC 48102 or ATCC 74340, preferably variants of M_thermophila, the sequence of which is shown at Fig. 7, said variants comprising at least one of the following amendments:
S24C; F31Y; H39S,Q; F40L,N; G42S;
   Q43A, C, D, E, F, G, H, I, K, L, M, N, P, R, S, T, V, W, Y; Y44N; S45D; Y47F; S49P; P51E,A,R; I56P; D58K,A; D59G; E61R; V62I; S69Q; A75W, F; L78D,S; K79G; R80K, A; A81Q, E, G; A82T; S83A, I, Q, K, R; Y84Q,V,I; R90A; D115N; E116S; T118V,L; R119E; T120L; Q122A; Q123N,T; M125S; V126H,S; N127Q,E; S128A,T; F132I,L; K143N; V148I; A151S; Q153D,Y; D154Q,S,G; H158D,A; S159T; A160S; E161T,N; G170fH; S170gA; T171N; F163W; V172P; R173Q,S; P184Q,S; E185S; T186A,E,P; G187aS; T190P, A; N193S; D194S,T; L195T, V; A198N,V; E200G,V; E201D; G201a () ; P201b (); Y201c (); S201d (); T201e (); I201f () ; G202S, A; D203R,K,S; D203aV,T; A204Q, E, S, V; Q205E; T211L,V; P215A; V220N,L; N223D,H; A232T; D233E; V235Y,L,T; A236Y,N; L237F; M238L; P242S; E244D; A251eE, Q; R256D; E260A, H; R264I; A265Q; Q270Y,A,L,G; S271D,N; K273D; Y275F; Y278T,H; P280A; T287A; Q288L,I,F; F292Y; V293A; ()302R,H; P304A; N336S; D337T,Q,S,G; M338I; M339V, I; G340P, A; G343A, S, F, I, L; D349P; P352K; D360R; E362P; Y364W,F; A365V, L, S; A366D, V, S; S367K, A; W368K; A369I, L; A373S; A374S; R375H; I376M; E383kQ; E387P; G404A; M409R; T411K; L412R; E417R; F421Y; D431E.

Also disclosed is a new phytase which has been derived from a strain of Cladorrhinum, viz. C. foecundissimum. Accordingly, a polypeptide having phytase acitivity and which comprises SEQ ID NO:2 or the mature part (amino acids nos 16-495) thereof is described; or a polypeptide being at least 70, more preferably 75, 80, 85, 90, 95% homologous thereto; homology meaning similarity, preferably identity, and being determined using the program GAP and the settings as defined hereinabove. And disclosed is a DNA construct which encodes a polypeptide having phytase activity, said DNA construct comprising a DNA molecule which comprises SEQ ID NO:1 or nucleotides nos. 20-70 and 207-1560 thereof; or nucleotides nos. 20-70 and 207-1563 thereof; or nucleotides nos. 65-70 and 207-1560 thereof; or nucleotides nos. 65-70 and 207-1563 thereof; or a DNA construct or molecule which is at least 70, 75, 80, 85, 90, 95 % homologous to either of these nucleotide sequences; homology meaning similarity, preferably identity, and being determined using computer programs known in the art such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3. Also disclosed is a DNA construct which hybridizes with any of the above DNA sequences under the conditions mentioned hereinabove.

### EXAMPLES

### Example 1

### Phytase activity assay (FYT)

Phytase activity can be measured using the following assay:
10 µl diluted enzyme samples (diluted in 0.1 M sodium acetate, 0.01 % Tween20, pH 5.5) are added into 250 µl 5 mM sodium phytate (Sigma) in 0.1 M sodium acetate, 0.01 % Tween20, pH 5.5 (pH adjusted after dissolving the sodium phytate; the substrate is preheated) and incubated for 30 minutes at 37°C. The reaction is stopped by adding 250 µl 10 % TCA and free phosphate is measured by adding 500 µl 7.3 g FeSO4 in 100 ml molybdate reagent (2.5 g (NH₄)₆Mo₇O₂₄.4H₂O in 8 ml H₂SO₄ diluted to 250 ml). The absorbance at 750 nm is measured on 200 µl samples in 96 well microtiter plates. Substrate and enzyme blanks are included. A phosphate standard curve is also included (0-2 mM phosphate). 1 FYT equals the amount of enzyme that releases 1 µmol phosphate/min at the given conditions.

### Example 2

### Test for specific activity

The specific activity can be determined as follows:

A highly purified sample of the phytase is used (the purity is checked beforehand on an SDS poly acryl amide gel showing the presence of only one component).

The protein concentration in the phytase sample is determined by amino acid analysis as follows: An aliquot of the phytase sample is hydrolyzed in 6N HCl, 0.1% phenol for 16 h at 110 C in an evacuated glass tube. The resulting amino acids are quantified using an Applied Biosystems 420A amino acid analysis system operated according to the manufacturers instructions. From the amounts of the amino acids the total mass - and thus also the concentration - of protein in the hydrolyzed aliquot can be calculated.

The activity is determined in the units of FYT. One FYT equals the amount of enzyme that liberates 1 micromol inorganic phosphate from phytate (5 mM phytate) per minute at pH 5.5, 37°C; assay described e.g. in example 1.

The specific activity is the value of FYT/mg enzyme protein.

### Example 3

### Test for temperature and pH activity and stability

Temperature and pH activity and stability can be determined as follows:
Temperature profiles (i.e. temperature activity relationship) by running the FYT assay of Example 1 at various temperatures (preheating the substrate).
Temperature stability by pre-incubating the phytase in 0.1 M sodium phosphate, pH 5.5 at various temperatures before measuring the residual activity.

The pH-stability by incubating the enzyme at pH 3 (25 mM glycine-HCl), pH 4-5 (25 mM sodium acetate), pH 6 (25 mM MES), pH 7-9 (25 mM Tris-HCl) for 1 hour at 40°C, before measuring the residual activity.

The pH-profiles (i.e. pH activity relationship) by running the assay at the various pH using the same buffer-systems (50 mM, pH re-adjusted when dissolving the substrate).

### Example 4

### DSC as a preferred test for thermostability

The thermostability or melting temperature, Tm, can be determined as follows:

In DSC the heat consumed to keep a constant temperature increase in the sample-cell is measured relative to a reference cell. A constant heating rate is kept (e.g. 90°C/hour). An endo-thermal process (heat consuming process - e.g. the unfolding of an enzyme/protein) is observed as an increase in the heat transferred to the cell in order to keep the constant temperature increase.

DSC can be performed using the MC2-apparatus from MicroCal. Cells are equilibrated 20 minutes at 20°C before scanning to 90°C at a scan rate of 90°/h. Samples of e.g. around 2.5 mg/ml phytase in 0.1 M sodium acetate, pH 5.5 are loaded.

### Example 5

### Phytase variants of amended activity characteristics

Variants of an Aspergillus fumigatus model phytase (a wild type phytase derived from strain ATCC 13073) were prepared as described in EP 98104858.0 (EP-A-0897010), examples 2-3 and 5, and the phytase activity was determined as described in example 7 thereof. pH- and temperature optimum and melting point was determined as described in examples 9 and 10 of EP 98113176.6 (EP-A-0897985).

In Table 1, variants of improved specific activity at pH 5.0 are listed. Table 2 lists variants of improved relative activity at pH 3.0, and Table 3 lists variants of improved thermostability (temperature optimum, e.g. determined by DSC).

**Table 1**

| **Amended in position no.** | **Substitution into** | **Specific activity at pH 5.0 (U/mg)** |
|---|---|---|
| 43 | 43L | 83.4 |
| | 43N | 45.5 |
| | 43T | 106.9 |
| | 43I | 91.2 |
| | 43V | 35.0 |
| | 43A | 27.3 |
| | 43G | 59.6 |
| 43 and 270 | 43L, 270L | 88.7 |
| 43 and 270 and 273 | 43L, 270L, 273D | 92.3 |
| 43 and 78 | 43L, 78D | 118.5 |
| 43 and 153 and 154 | 43L, 153Y, 154G | 193.0 |
| A. fumigatus wild-type phytase | - | 26.5 |

**Table 2**

| **Amended in position no.** | **Substitution into** | **Relative phytase activity at pH 3.0** |
|---|---|---|
| 205 | 205E | 41% |
| 273 | 273K | 61% |
| 278 | 278H | 75% |
| 273 and 205 | 273K, 205E | 65% |
| 273 and 278 | 273K, 278H | 100% |
| 273 and 205 and 278 | 273K, 205E, 278H | 96% |
| A. fumigatus wild-type phytase | - | 32% |

**Table 3**

| **Amended in position no.** | **Substitution into** | **Temperature optimum (°C)** | **Tm (°C) (DSC)** |
|---|---|---|---|
| 43 and 47 and 88 and 102 and 220 and 242 and 267 | 43T, 47Y, 88I, 102Y, 220L, 242P, 267D | 60 | 67 |
| as above plus 51 and 302 and 337 and 373 and 115 | as above plus 51A, 302H, 337T, 373A, 115N | 63 | - |
| A. fumigatus wild-type phytase | - | 55 | 62.5 |

### Example 6

### Further phytase variants of amended activity characteristics

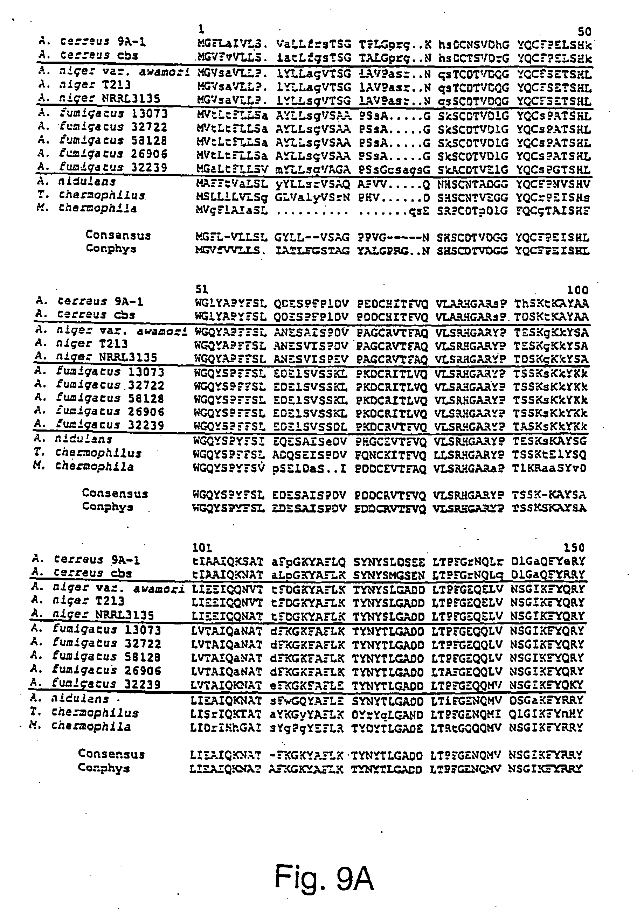

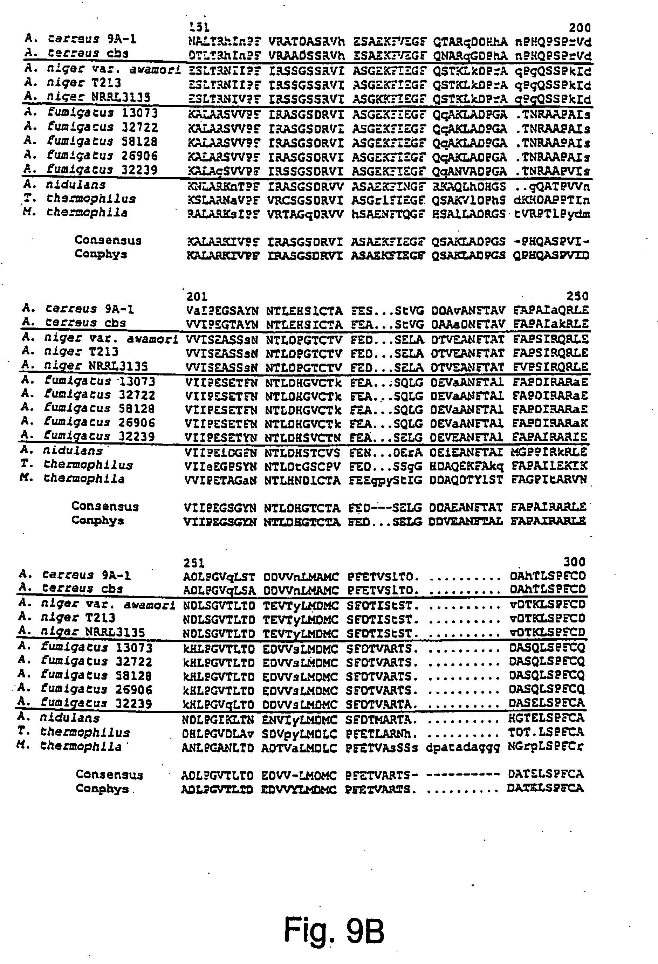

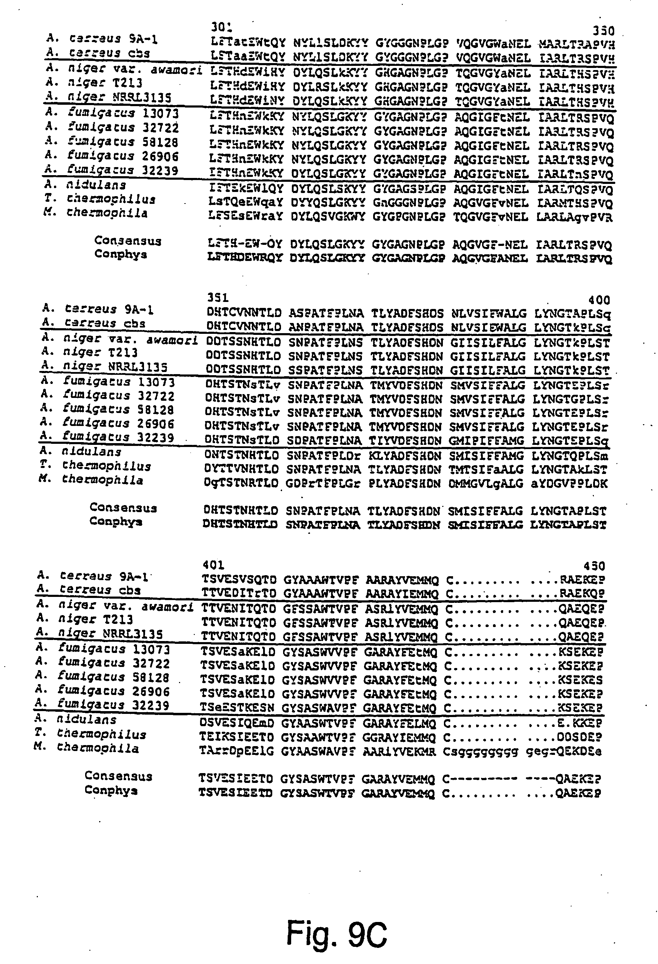

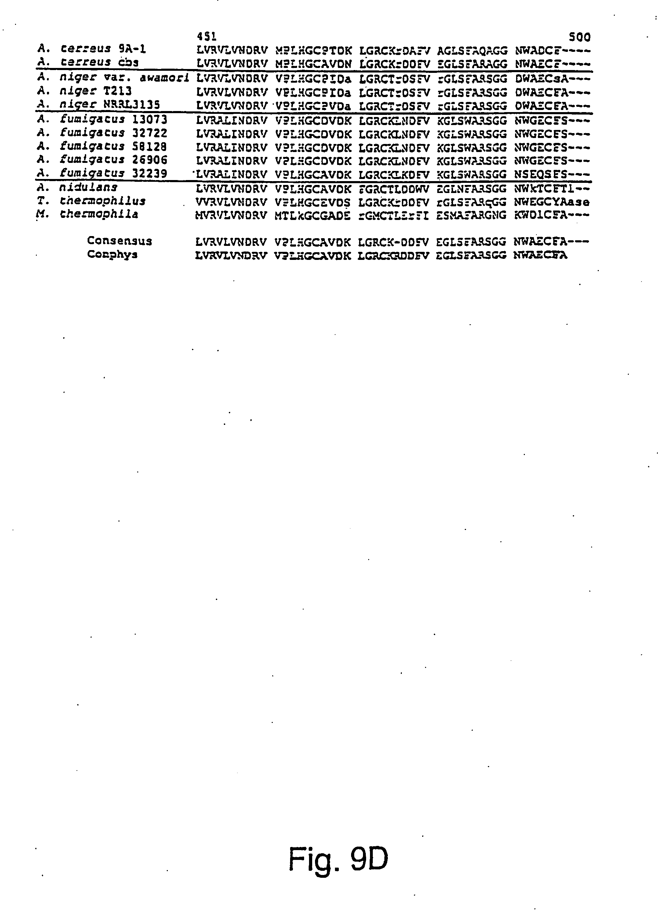

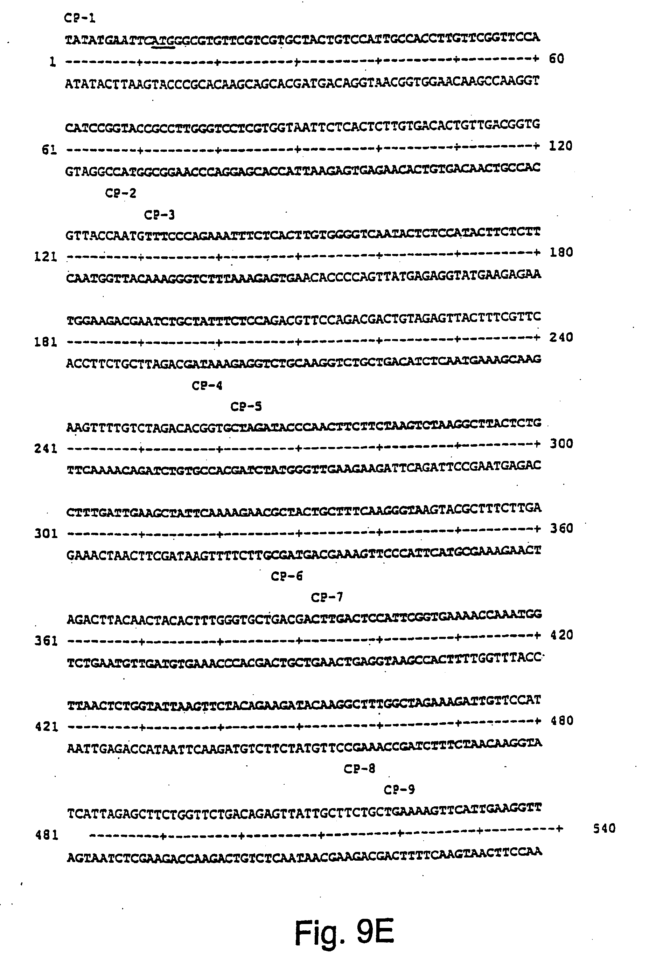

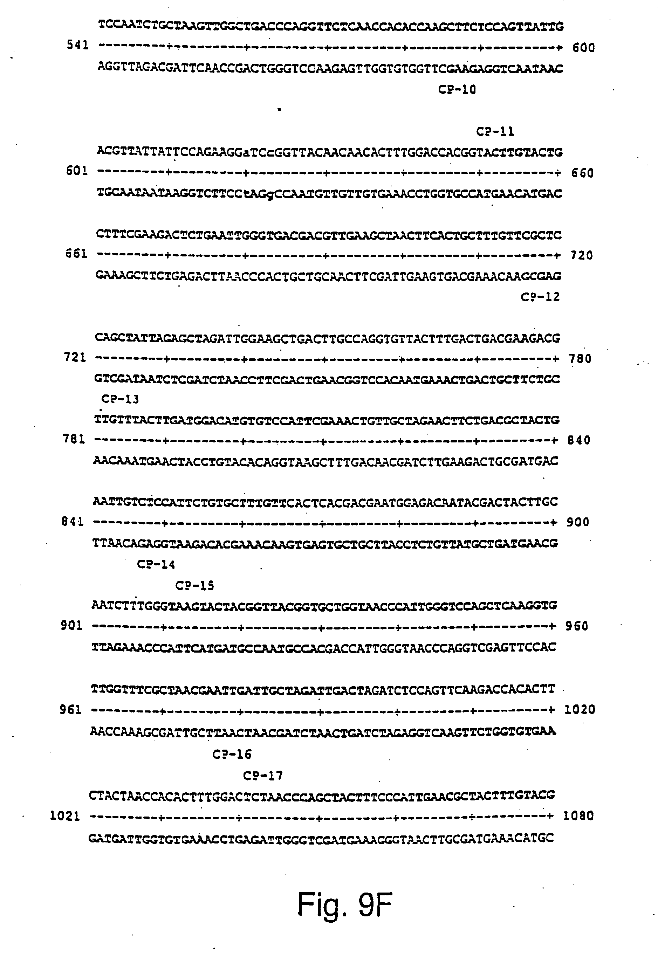

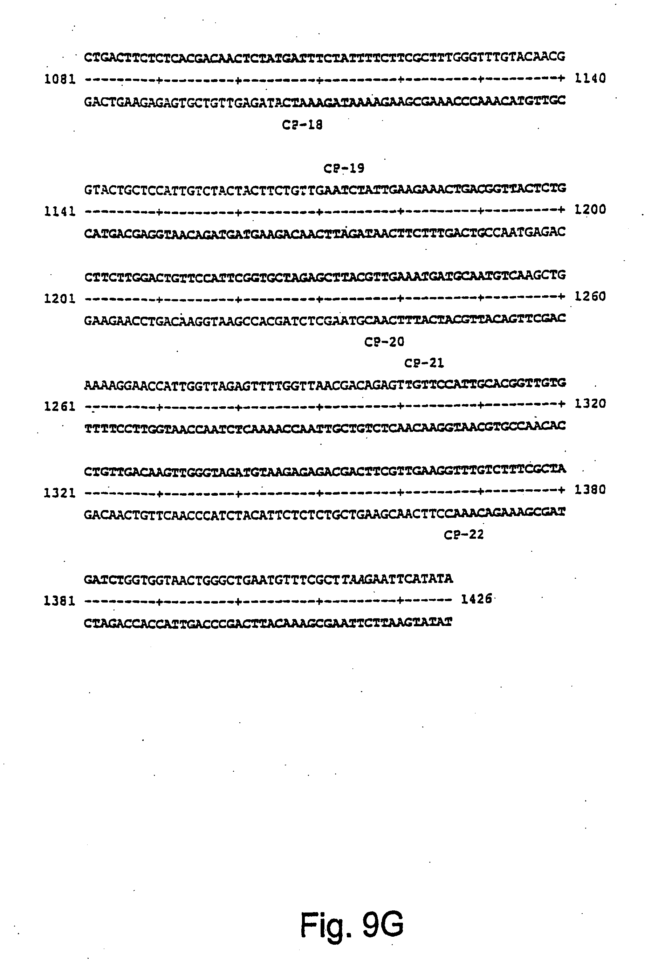

Variants of the ascomycete consensus sequence "conphys" of Fig. 9 were prepared as described in EP 98113176.6 (EP-A-0897985), examples 4-8. Phytase activity, including pH- and temperature optimum, and melting point was determined as described in examples 9 and 10, respectively, thereof.

The tables below list variants of amended activity characteristics, viz.

Table 4 variants of improved specific activity at pH 6.0;

Table 5 variants of amended pH optimum (the pH-optimum indicated is an approximate value, determined as that pH-value (selected from the group consisting of pH 4.0; 4.5; 5.0; 5.5; 6.0; 6.5; and 7;0) at which the maximum phytase activity was obtained);

Table 6 a variant of improved thermostability (expressed by way of the melting point as determined by differential scanning calorimetry (DSC)); and

Table 7 variants of amended thermostability (temperature optimum); a "+" or "-" indicates a positive or a negative, respectively, effect on temperature optimum of up to 1°C; and a "++" and "- -" means a positive or a negative, respectively, effect on temperature optimum of between 1 and 3°C.

**Table 4**

| **Amended in position no.** | **Substitution into** | **Specific activity at pH 6.0 (U/mg)** |
|---|---|---|
| 43 | 43T | 130 |
| | 43L | 205 |
| Conphys | - | 62 |

**Table 5**

| **Amended in position no.** | **Substitution into** | **pH optimum around** |
|---|---|---|
| 43 | 43T | 6.0 |
| | 43L | 5.5 |
| | 43G | 6.5 |
| 43 and 44 | 43L, 44N | 6.0 |
| | 43T, 44N | 5.5 |
| Conphys | - | 6.0 |

**Table 6**

| **Amended in position no.** | **Substitution into** | **Tm (°C)** |
|---|---|---|
| 43 | 43T | 78.9 |
| Conphys | - | 78.1 |

**Table 7**

| **Amended in position no.** | **Substitution into** | **Temperature optimum amendment** |
|---|---|---|
| 51 | A | + |
| 58 | K | + |
| 220 | N | + |
| 19.5 | L | ++ |
| 201e | T | ++ |
| 244 | D | + |
| 264 | I | + |
| 302 | H | + |
| 337 | T | ++ |
| 352 | K | + |
| 373 | A | ++ |
| 47 | F | - |
| 62 | I | - |
| 83 | K | - |
| 90 | R | - |
| 143 | N | - |
| 148 | V | -- |
| 186 | A | -- |
| 187a | S | - |
| 198 | V | - |
| 204 | A | -- |
| 211 | V | - |
| 215 | P | -- |
| 251e | Q | - |
| 260 | A | - |
| 265 | A | - |
| 339 | V | - |
| 365 | A | -- |
| 383k | E | - |
| 404 | G | -- |
| 417 | R | -- |
| Conphys | - | 0 |

**Table 8**

| **Amended in position no.** | **Substitution into** | **Tm (°C) (DSC)** | **Specific activity at pH 5.0 (U/mg)** |
|---|---|---|---|
| 43 and 51 and 220 and 244 and 264 and 302 and 337 and 352 and 373 | 51A, 220N, 244D, 264I, 302H, 337T, 352K, 373A, 43T | 84.7 | 105 |
| as above plus 80 | as above plus 80A | 85.7 | 180 |
| Conphys | - | 78.1 | 30 |

### Example 7

### Cloning of a phytase of Cladorrhinum foecundissimum

DNA encoding a phytase from Cladorrhinum foecundissimum CBS 427.97 has been cloned, and the enzyme isolated and purified, essentially as described in WO 98/28409.

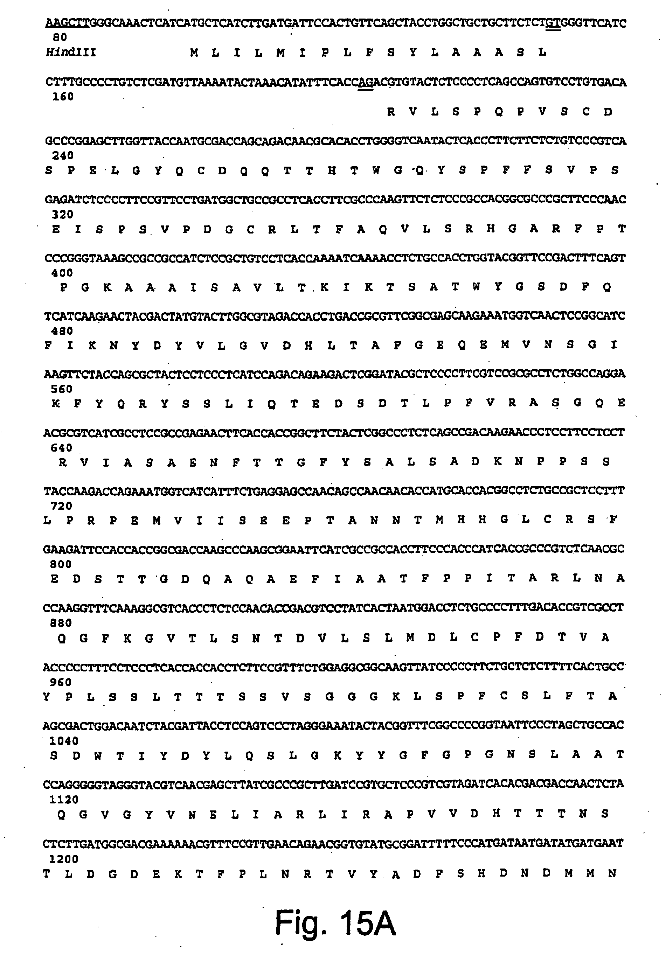

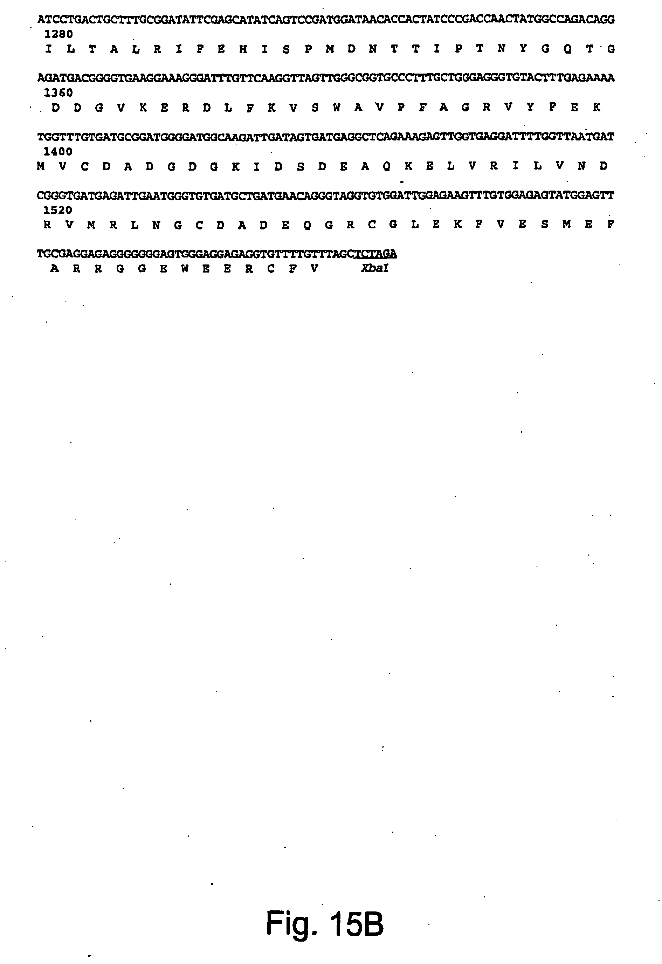

Fig. 15 shows the DNA sequence of the HindIII/XbaI cloned PCR product in pA2phy8. The cloned PCR product is amplified from the genomic region encoding Cladorrhinum foecundissimum CBS 427.97 phyA gene. The putative intron is indicated by double underline of the excision-ligation points in accordance with the GT-AG rule (R. Breathnach et al. Proc. Natl. Acad. Sci. USA 75 (1978) pp4853-4857). The restrictions sites used for cloning are underlined.

According to the SignalP V1.1 prediction (Henrik Nielsen, Jacob Engelbrecht, Stren Brunak and Gunnar von Heijne: "Identification of prokaryotic and eukaryotic signal peptides and prediction of their cleavage sites," Protein Engineering 10, 1-6 (1997)), the signal peptide part of the enzyme corresponds to amino acids nos. 1-15, accordingly the mature enzyme is amino acids nos. 16-495.

The enzyme exhibits a pH optimum around pH 6 with no activity at the low pH (pH 3), but significant activity up until pH 7.5; thus it is a more alkaline phytase as compared to the Aspergillus ficuum phytase.

A temperature optimum around 60°C was found at pH 5.5. Thus, this phytase is more thermostable than the A. *ficuum* phytase.

### Example 8

### Alignment of a new model phytase according to Fig. 1

The phytase sequence of Cladorrhinum foecundissimum as disclosed in Example 7 is compared with the 13 model phytases of Fig. 1 using GAP version 8 referred to above with a GAP weight of 3.000 and a GAP lengthweight of 0.100. Complete amino acid sequences are compared. The M thermophila phytase sequence turns up to be the most homologous sequence, showing a degree of similarity to the C. foecundissimum sequence of 70.86%.

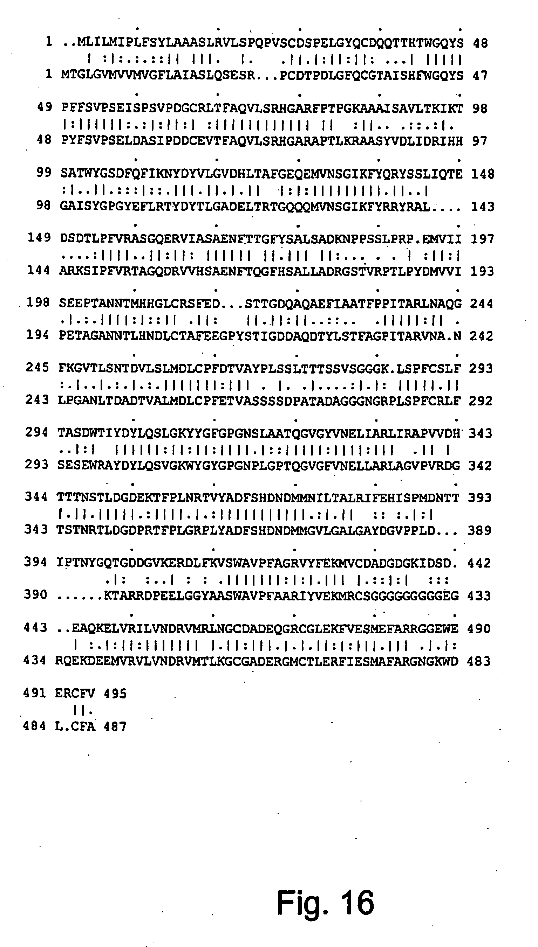

Still using the GAP program and the parameters mentioned above, the phytase sequence "C foecundissimum" is now aligned to the "M-thermophila" phytase - see Fig. 16. The average match is 0.540;, the average mismatch -0.396; quality 445.2; length 505; ratio 0.914; gaps 9; percent similarity 70.860; percent identity 53.878.

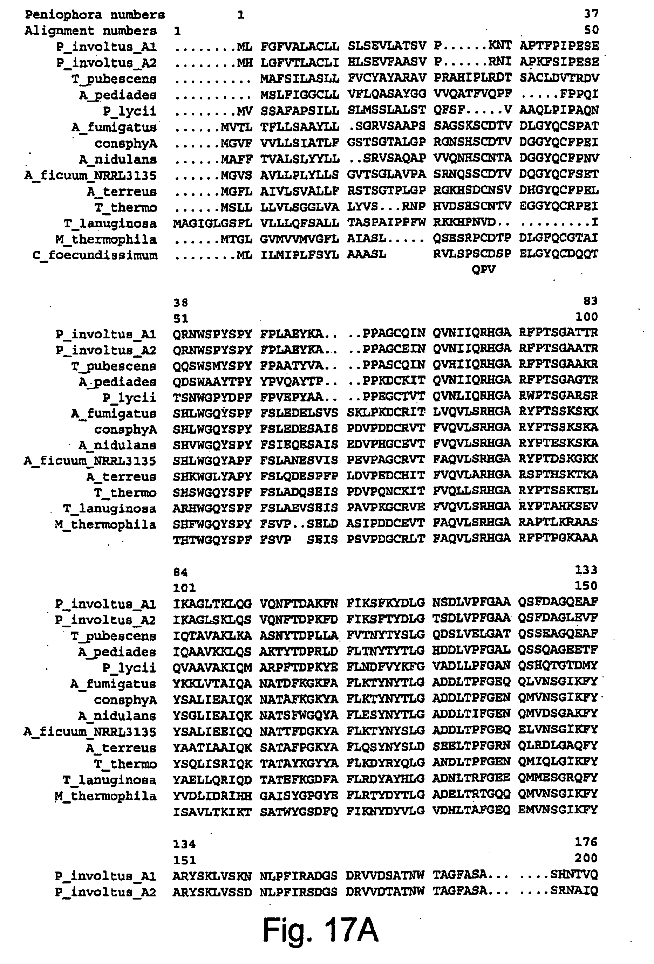

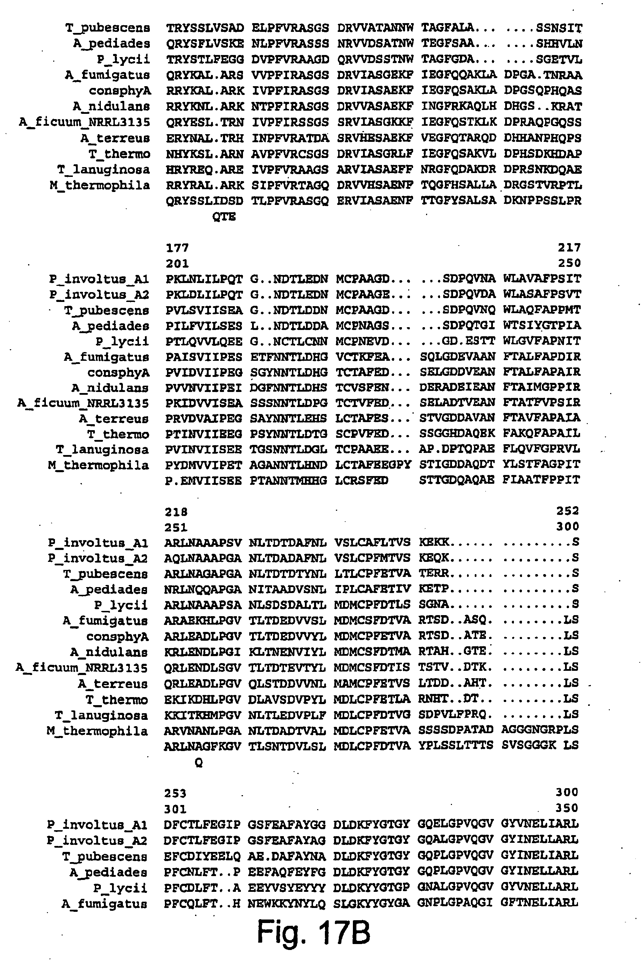

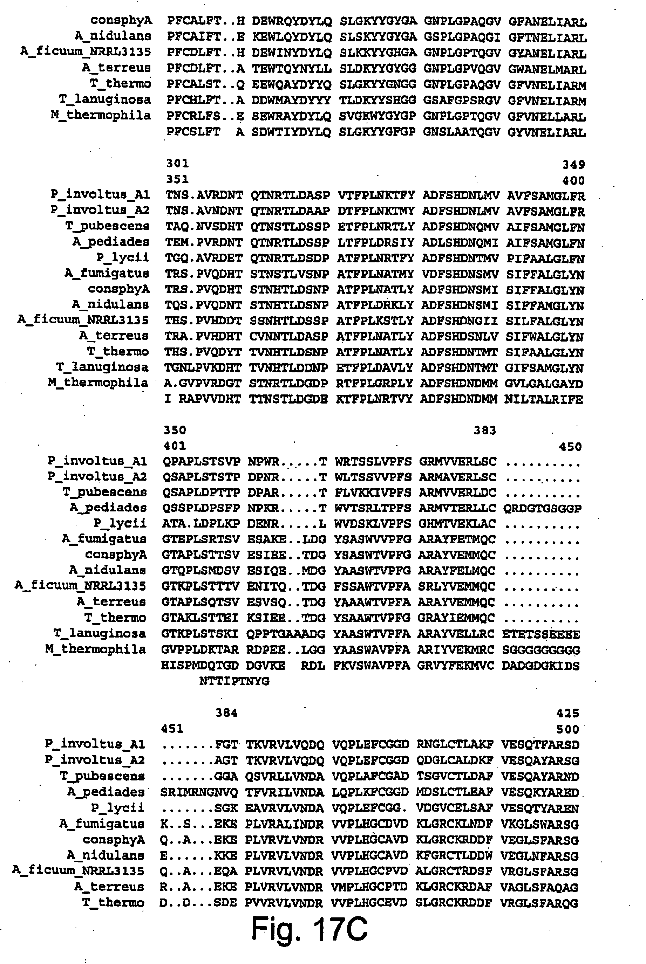

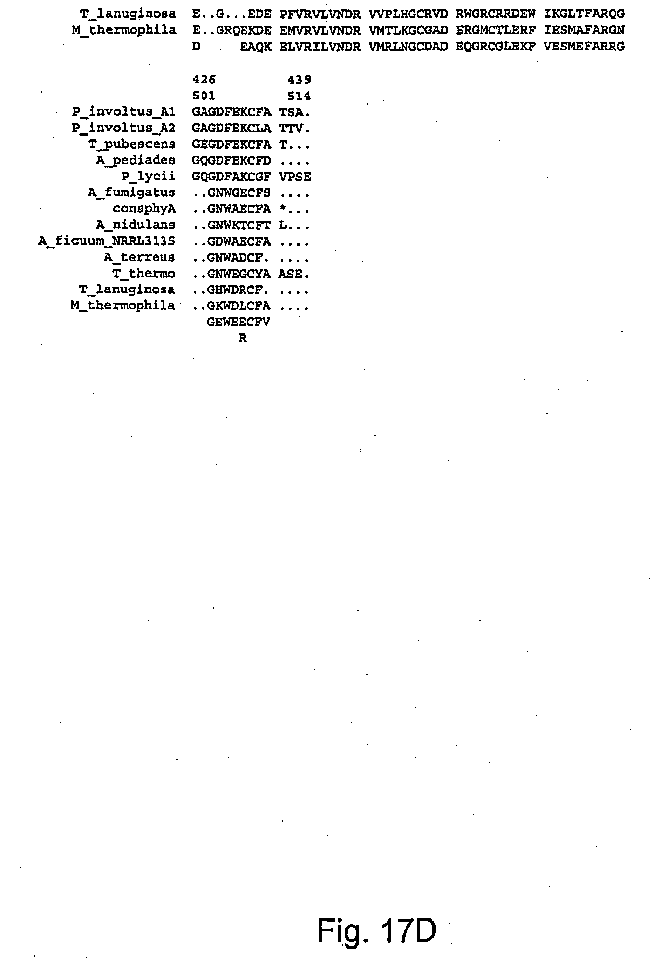

In a next step, see Fig. 17, the C_foecundissimum is pasted (or it could simply be written) onto the alignment of Fig. 1 as the bottom row, ensuring that those amino acid residues which according to the alignment at Fig. 16 are identical (indicated by a vertical line) or similar (indicated by one or two dots) are placed above each other. At 5 places along the sequence, the C_foecundissimum sequence comprises "excess" amino acid residues, which the alignment of Fig. 1 does not make room for. At Fig. 17, these excess residues are transferred onto a next row (but they can be included in the multiple alignment and numbered as described previously in the position numbering related paragraphs (using the denotations a, b, c etc.).

Corresponding variants of the phytase of C_foecundissimum are then easily deduced on the basis of Fig. 17. Some examples: The variants generally designated "80K,A" and "43T" in C_foecundissimum correspond to "K80A" and "Q43T," respectively.

### SEQUENCE LISTING

<212> DNA
   <213> Cladorrhinum foecundissimum
<220>
   <221> intron
   <222> (71)..(126)
<220>
   <221> CDS
   <222> (20)..(70)
<220>
   <221> CDS
   <222> (127)..(1563)
<220>
   <221> sig_peptide
   <222> (20)..(64)
<400> 1
<210> 2
   <211> 495
   <212> PRT
   <213> Cladorrhinum foecundissimum
<400> 2
   Met Leu Ile Leu Met Ile Pro Leu Phe Ser Tyr Leu Ala Ala Ala Ser
   1               5                   10                  15
   Leu Arg Val Leu Ser Pro Gln Pro Val Ser Cys Asp Ser Pro Glu Leu
               20                  25                  30
   Gly Tyr Gln Cys Asp Gln Gln Thr Thr His Thr Trp Gly Gln Tyr Ser
           35                  40                  45
   Pro Phe Phe Ser Val Pro Ser Glu Ile Ser Pro Ser Val Pro Asp Gly
       50                  55                  60
   Cys Arg Leu Thr Phe Ala Gln Val Leu Ser Arg His Gly Ala Arg Phe
   65                  70                  75                  80
   Pro Thr Pro Gly Lys Ala Ala Ala Ile Ser Ala Val Leu Thr Lys Ile
                   85                  90                  95
   Lys Thr Ser Ala Thr Trp Tyr Gly Ser Asp Phe Gln Phe Ile Lys Asn
               100                 105                 110
   Tyr Asp Tyr Val Leu Gly Val Asp His Leu Thr Ala Phe Gly Glu Gln
           115                 120                 125
   Glu Met Val Asn Ser Gly Ile Lys Phe Tyr Gln Arg Tyr Ser Ser Leu
       130                 135                 140
   Ile Gln Thr Glu Asp Ser Asp Thr Leu Pro Phe Val Arg Ala Ser Gly
   145                 150                 155                 160
   Gln Glu Arg Val Ile Ala Ser Ala Glu Asn Phe Thr Thr Gly Phe Tyr
                   165                 170                 175
   Ser Ala Leu Ser Ala Asp Lys Asn Pro Pro Ser Ser Leu Pro Arg Pro
               180                 185                 190
   Glu Met Val Ile Ile Ser Glu Glu Pro Thr Ala Asn Asn Thr Met His
           195                 200                 205
   His Gly Leu Cys Arg Ser Phe Glu Asp Ser Thr Thr Gly Asp Gln Ala
       210                 215                 220
   Gln Ala Glu Phe Ile Ala Ala Thr Phe Pro Pro Ile Thr Ala Arg Leu
   225                 230                 235                 240
   Asn Ala Gln Gly Phe Lys Gly Val Thr Leu Ser Asn Thr Asp Val Leu
                   245                 250                 255
   Ser Leu Met Asp Leu Cys Pro Phe Asp Thr Val Ala Tyr Pro Leu Ser
               260                 265                 270
   Ser Leu Thr Thr Thr Ser Ser Val Ser Gly Gly Gly Lys Leu Ser Pro
           275                 280                 285
   Phe Cys Ser Leu Phe Thr Ala Ser Asp Trp Thr Ile Tyr Asp Tyr Leu
       290                 295                 300
   Gln Ser Leu Gly Lys Tyr Tyr Gly Phe Gly Pro Gly Asn Ser Leu Ala
   305                 310                 315                 320
   Ala Thr Gln Gly Val Gly Tyr Val Asn Glu Leu Ile Ala Arg Leu Ile
                   325                 330                 335
   Arg Ala Pro Val Val Asp His Thr Thr Thr Asn Ser Thr Leu Asp Gly
                   340                 345                 350
   Asp Glu Lys Thr Phe Pro Leu Asn Arg Thr Val Tyr Ala Asp Phe Ser
           355                 360                 365
   His Asp Asn Asp Met Met Asn Ile Leu Thr Ala Leu Arg Ile Phe Glu
       370                 375                 380
   His Ile Ser Pro Met Asp Asn Thr Thr Ile Pro Thr Asn Tyr Gly Gln
   385                 390                 395                 400
   Thr Gly Asp Asp Gly Val Lys Glu Arg Asp Leu Phe Lys Val Ser Trp
                   405                 410                 415
   Ala Val Pro Phe Ala Gly Arg Val Tyr Phe Glu Lys Met Val Cys Asp
               420                 425                 430
   Ala Asp Gly Asp Gly Lys Ile Asp Ser Asp Glu Ala Gln Lys Glu Leu
           435                 440                 445
   Val Arg Ile Leu Val Asn Asp Arg Val Met Arg Leu Asn Gly Cys Asp
       450                 455                 460
   Ala Asp Glu Gln Gly Arg Cys Gly Leu Glu Lys Phe Val Glu Ser Met
   465                 470                 475                 480
   Glu Phe Ala Arg Arg Gly Gly Glu Trp Glu Glu Arg Cys Phe Val
                   485                 490                 495

## Claims

1. A phytase which differs from the Peniophora lycii phytase amino acid sequence of Fig. 6 in one or more of the following positions, using the position numbering corresponding to P_lycii of Fig. 1: D45S; T61R; A115N; L118V; H126S,V; G172P; E173Q,S; D201E; D203R, K, S; N215A, P; M238L; V264R, I; G302R,H; T337Q,S,G; V339I; P340A; E360R; V365L,A,S; D366V,S; E411K,T.

2. A phytase polypeptide which comprises a phytase according to claim 1.

3. A DNA construct comprising a DNA sequence encoding a phytase according to any one of claims 1-2.

4. A recombinant expression vector which comprises a DNA construct according to claim 3.

5. A host cell which is transformed with a DNA construct according to claim 3 or a vector according to claim 4.

6. A process for preparing a phytase, the process comprising culturing the host cell according to claim 5 under conditions permitting the production of the phytase, and recovering the phytase from the culture broth.

7. A feed or food comprising at least one phytase of any of claims 1-2.

8. A process for preparing a feed or food according to claim 7, wherein the at least one phytase is added to the food or feed components.

9. A composition comprising at least one phytase of any of claims 1-2.

10. The composition according to claim 9 suitable for use in food or feed preparations.

11. The composition according to any of claims 9-10 which is an animal feed additive.

12. A process for reducing phytate levels in animal manure comprising feeding an animal with an effective amount of the feed according to claim 7 or obtainable according to claim 8.

13. Use of the phytase of any of claims 1-2; or the composition of any of claims 9-10 for liberating phosphorus from a phytase substrate.

14. A transgenic plant or plant part which is capable of expressing a phytase according to any one of claims 1-2.

## Patentansprüche

1. Phytase, welche sich von der Aminosäuresequenz der Peniophora-lycii-Phytase von Fig. 6 in einer oder mehreren der folgenden Positionen unterscheidet, unter Verwendung der Nummerierung der Positionen entsprechend zu P_lycii von Fig. 1: D45S; T61R; A115N; L118V; H126S,V; G172P; E173Q,S; D201E; D203R,K,S; N215A,P; M238L; V264R,I; G302R,H; T337Q,S,G; V339I; P340A; E360R; V365L,A,S; D366V,S; E411K,T.

2. Phytasepolypeptid, welches eine Phytase gemäß Anspruch 1 umfasst.

3. DNA-Konstrukt, umfassend eine DNA-Sequenz, die eine Phytase gemäß einem beliebigen der Ansprüche 1-2 kodiert.

4. Rekombinanter Expressionsvektor, welcher ein DNA-Konstrukt gemäß Anspruch 3 umfasst.

5. Wirtszelle, welche mit einem DNA-Konstrukt gemäß Anspruch 3 oder einem Vektor gemäß Anspruch 4 transformiert ist.

6. Verfahren zum Herstellen einer Phytase, wobei das Verfahren das Kultivieren der Wirtszelle gemäß Anspruch 5 unter Bedingungen, die die Herstellung der Phytase erlauben, und das Rückgewinnen der Phytase aus der Kulturbrühe umfasst.

7. Futter oder Nahrungsmittel, umfassend mindestens eine Phytase gemäß einem beliebigen der Ansprüche 1-2.

8. Verfahren zum Zubereiten eines Futters oder Nahrungsmittels gemäß Anspruch 7, wobei die mindestens eine Phytase zu den Futter- oder Nahrungsmittelbestandteilen hinzugefügt wird.

9. Zusammensetzung, umfassend mindestens eine Phytase nach einem beliebigen der Ansprüche 1-2.

10. Zusammensetzung nach Anspruch 9, geeignet zur Verwendung in Futter- oder Nahrungsmittelzubereitungen.

11. Zusammensetzung nach einem beliebigen der Ansprüche 9-10, welche ein Tierfutteradditiv ist.

12. Verfahren zum Reduzieren von Phytatspiegeln in Tierdung, umfassend das Füttern eines Tieres mit einer wirksamen Menge des Futters gemäß Anspruch 7, oder eines Futters erhältlich gemäß Anspruch 8.

13. Verwendung der Phytase gemäß einem beliebigen der Ansprüche 1-2; oder der Zusammensetzung gemäß einem beliebigen der Ansprüche 9-10 zum Freisetzen von Phosphor aus einem Phytasesubstrat.

14. Transgene Pflanze oder Pflanzenteil, welche(r) zum Exprimieren einer Phytase gemäß einem beliebigen der Ansprüche 1-2 fähig ist.

## Revendications

1. Phytase qui diffère de la séquence en acides aminés de la phytase de Peniophora lycii de la Fig.6 en une ou plusieurs des positions suivantes, lorsque l'on utilise la numérotation de position correspondant à P. lycci de la Fig. 1 : D45S ; T61R ; A115N ; L118V ; H126S,V ; G172P ; E173Q,S ; D201E ; D203R,K,S ; N215A,P ; M238L ; V264R,I ; G302R,H ; T337Q,S,G ; V339I ; P340A ; E360R ; V365L,A,S ; D366V,S ; E411K,T.

2. Polypeptide de phytase qui comprend une phytase selon la revendication 1.

3. Construction d'ADN comprenant une séquence d'ADN codant une phytase selon l'une quelconque des revendications 1 à 2.

4. Vecteur d'expression de recombinant qui comprend une construction d'ADN selon la revendication 3.

5. Cellule hôte qui est transformée avec une construction d'ADN selon la revendication 3 ou un vecteur selon la revendication 4.

6. Procédé pour préparer une phytase, ledit procédé comprenant la mise en culture de la cellule hôte selon la revendication 5 dans des conditions permettant la production de la phytase, et la récupération de la phytase à partir du bouillon de culture.

7. Aliment comprenant au moins une phytase selon l'une quelconque des revendications 1 à 2.

8. Procédé pour préparer un aliment selon la revendication 7, dans lequel la au moins une phytase est ajoutée aux composants de l'aliment.

9. Composition comprenant au moins une phytase de l'une quelconque des revendications 1 à 2.

10. Composition selon la revendication 9 adaptée à l'usage dans des préparations alimentaires.

11. Composition selon l'une quelconque des revendications 9 à 10 qui est un additif d'aliment pour animaux.

12. Procédé pour réduire des niveaux de phytate dans de l'engrais animal comprenant l'alimentation d'un animal avec une quantité efficace de l'aliment selon la revendication 7 ou susceptible d'être obtenu selon la revendication 8.

13. Utilisation de la phytase selon l'une quelconque des revendications 1 à 2 ; ou composition selon l'une quelconque des revendications 9 à 10 pour libérer le phosphore à partir d'un substrat de phytase.

14. Plante transgénique ou partie de plante qui est capable d'exprimer une phytase selon l'une quelconque des revendications 1 à 2.
